# EUROPEAN PATENT APPLICATION

(11) **EP 4 039 268 A1**
(43) Date of publication of application: **10.08.2022**
(21) Application number: 21155227.8
(22) Date of filing: 04.02.2021
(51) Int. Cl.: A61K 38/17, G01N 33/68

(54) **TREATMENT AND METHOD OF IDENTIFYING CORONAVIRUS THERAPEUTICS**

(71) Applicant: IMBA-Institut für Molekulare Biotechnologie GmbH, 1030 Wien (AT)
(72) Inventor: HOFFMANN, David, 1030 Wien (AT); MEREITER, Stefan, 1030 Wien (AT); PENNINGER, Josef, 1130 Wien (AT); WIRNSBERGER, Gerald, 1030 Wien (AT)
(74) Representative: SONN Patentanwälte OG

(57) **Abstract**

The invention provides a polypeptide or nucleic acid for use in a method of treating a coronavirus infection comprising administering a polypeptide comprising a carbohydrate recognition domain of CLEC4G or a nucleic acid encoding said polypeptide to a patient suffering from an infection with a coronavirus expressing a SARS-CoV-2 Spike protein.

## Description

The present invention relates to the field of coronavirus therapeutics and the identification of therapeutic agents.

### Background of the invention

COVID-19 caused by SARS-CoV-2 infections has triggered a pandemic massively disrupting health care, social and economic life. SARS-CoV-2 entry into target cells is mediated by the viral Spike protein, which binds to angiotensin converting enzyme 2 (ACE2) expressed on host cells (Monteil et al. (2020) Cell 181, 905-913 e907). The Spike protein is divided into two subunits, S1 and S2. The S1 subunits comprises the receptor binding domain (RBD) which confers ACE2 binding activity. The S2 subunit mediates virus fusion with the cell wall following proteolytic cleavage (Hoffmann et al. (2020) Cell 181, 271-280 e278; Shang et al. (2020) Proc Natl Acad Sci USA 117, 11727-11734; Walls et al. (2020) Cell 181, 281-292 e286). Cryo-electron microscopy studies have shown that the Spike protein forms a highly flexible homotrimer containing 22 *N*-glycosylation sites each (Walls et al., 2020, *supra*).

Glycosylation of viral proteins ensures proper folding and shields antigenic viral epitopes from immune recognition (Watanabe et al. (2019) Biochim Biophys Acta Gen Subj 1863, 1480-1497; Watanabe et al. (2020) Nat Commun 11, 2688). To create this glycan shield, the virus hijacks the host glycosylation machinery and thereby ensures the presentation of self-associated glycan epitopes. Apart from shielding epitopes from antibody recognition, glycans can be ligands for lectin receptors. For instance, mannose-specific mammalian lectins, like DC-SIGN (CD209) or its homolog L-SIGN (CD299), bind to viruses like HIV-1 (Van Breedam et al. (2014) FEMS Microbiol Rev 38, 598-632). Lectin receptors are often expressed on immune and endothelial cells and serve as pattern recognition receptors involved in virus internalization and transmission (Osorio et al. (2011) Immunity 34, 651-664). Recent studies have characterized the recognition of the SARS-CoV-2 Spike by previously known virus-binding lectins, such as DC-SIGN, L-SIGN, MGL and MR (Gao et al. (2020) bioRxiv, doi.org/10.1101/2020.07.29.227462). Given that SARS-CoV-2 relies less on oligo-mannose-type glycosylation, as compared to for instance HIV-1, and displays more complex-type glycosylation, it is unknown if additional lectin receptors are capable of binding the Spike protein and whether such interactions might have functional relevance in SARS-CoV-2 infections.

There remains a need to find effective therapies for corona-virus infections, especially SARS-CoV-2 infections. It is therefore a goal of the invention to find new therapies for corona-virus infections and in particular for COVID-19.

### Summary of the invention

The present invention provides a polypeptide or nucleic acid for use in a method of treating a coronavirus infection comprising administering a polypeptide comprising a carbohydrate recognition domain of CLEC4G or a nucleic acid encoding said polypeptide to a patient suffering from an infection with a coronavirus expressing a SARS-CoV-2 Spike protein.

Related thereto, the invention provides a method of treating a coronavirus infection comprising administering a polypeptide comprising a carbohydrate recognition domain of CLEC4G or a nucleic acid encoding said polypeptide to a patient suffering from an infection with a coronavirus expressing a SARS-CoV-2 Spike protein.

Also provided is a use of a polypeptide or nucleic acid for manufacturing a medicament for a treatment of a patient suffering from an infection with a coronavirus expressing a SARS-CoV-2 Spike protein, the treatment comprising administering a polypeptide comprising a carbohydrate recognition domain of CLEC4G or a nucleic acid encoding said polypeptide to a patient suffering from an infection with a corona-virus expressing a SARS-CoV-2 Spike protein.

Further provided is a library of at least 10 different polypeptides, each polypeptide comprising a different carbohydrate recognition domain of a lectin and an immunoglobulin domain.

The invention also provides a set of nucleic acids encoding the polypeptides of the library.

Also provided is a method of identifying or screening a lectin candidate that binds to a carbohydrate-containing target of interest comprising contacting the target with the polypeptides of the library, and detecting polypeptides bound to said target.

All aspects of the invention are related and any disclosure of specific embodiments for one aspect also relate to other aspects. E.g. a disclosure of polypeptides of the library equally relates to nucleic acids encoding said polypeptides. Such polypeptides and nucleic acids can be used in the inventive methods.

### Detailed description of the invention

Most viruses use glycosylation for pathogenesis and immune evasion with critical implications for therapies or vaccinations. Here we used a comprehensive library of mammalian carbohydrate-binding proteins (lectins) to probe critical sugar residues on the full-length trimeric Spike and the receptor binding domain (RBD) of SARS-CoV-2. Two lectins, Clec4g and CD209, were identified to strongly bind to Spike. Clec4g and CD209 binding to Spike was dissected and visualized in real time and at single molecule resolution using atomic force microscopy. 3D modelling showed that Clec4g can bind to a glycan within the RBD-ACE2 interface and thus interferes with Spike binding to cell surfaces. Importantly, Clec4g and CD209 significantly reduced SARS-CoV-2 infections. The invention provides the first extensive map and 3D structural modelling of lectin-Spike interactions and uncover lectins critically involved in Spike binding and SARS-CoV-2 infections.

As Spike and RBD glycosylation affect ACE2 binding and SARS-CoV-2 infections, targeting virus specific glycosylation is a novel means for therapeutic intervention.

The present invention provides a polypeptide or nucleic acid for use in a method of treating a coronavirus infection comprising administering a polypeptide comprising a carbohydrate recognition domain of CLEC4G or a nucleic acid encoding said polypeptide to a patient suffering from an infection with a coronavirus expressing a SARS-CoV-2 Spike protein.

CLEC4G (C-Type Lectin Domain Family 4 Member G) is a lectin with its sequence being deposited at the UniProtKB database entry Q6UXB4 (Entry version 129 of 2 December 2020, provided as SEQ ID NO: 2). As used herein, it refers to any form, variant or origin of the protein. CLEC4G is preferably a mammalian protein, preferably a human protein. Other CLEC4G proteins are from mouse, rat, hamster, cow, cat, non-human primate, monkey, pig, horse, dog, etc. Other names of CLEC4G according to genecards.org are: C-Type Lectin Domain Family 4 Member G; LSECtin; Liver And Lymph Node Sinusoidal Endothelial Cell; C-Type Lectin Superfamily 4, Member G; UNQ431; C-Type Lectin Domain Family 4, Member G.

A preferred CLEC4G protein is human CLEC4G according to all embodiments of the invention and is used as reference (Liu et al., JBC 279(18), 2004: 18748-18758). Human CLEC4G (SEQ ID NO: 2) comprises at amino acids (aa) 1-31 a cytoplasmic domain, a transmembrane domain at aa 32-52, and at aa 53-293 an extracellular domain. The carbohydrate recognition domain of human CLEC4G is within the extracellular domain and may comprise amino acids 165-288 of SEQ ID NO: 2.

According to the invention, a polypeptide comprising a carbohydrate recognition domain of CLEC4G or a nucleic acid encoding said polypeptide is sufficient. Of course, the invention also includes variants thereof that are capable of binding SARS-CoV-2 Spike protein, such as the Spike protein of SEQ ID NO: 1. A preferred carbohydrate recognition domain of CLEC4G as used in the invention has at least 60% amino acid identity to the amino acids 165-288 of SEQ ID NO: 2 (human CLEC4G). In even more preferred embodiments, the carbohydrate recognition domain of CLEC4G has at least 65%, more preferred at least 70%, at least 75%, at least 80%, at least 85%, at least 90%, at least 95% or at least 98%, or at least 99% amino acid identity to the amino acids 165-288 of SEQ ID NO: 2. As shown in example 20, mouse CLEC4G, also termed Clec4g, is effective in preventing SARS-CoV-2 of human cells. Mouse CLEC4G is e.g. available in the UniProt database entry Q8BNX1 (entry version 128 of 2 December 2020, incorporated herein by reference). The CRD of human CLEC4G has a sequence identity of 70.5% to the CRD of mouse CLEC4G, clearly illustrating that sequence variation while maintaining binding to SARS-CoV-2 Spike protein and protecting against SARS-CoV-2 is possible.

Sequence identities are calculated with regard to a reference sequence, here in the above paragraph to the CRD of human CLEC4G of SEQ ID NO: 2. The compared test sequence (in the above example mouse CLEC4G) by aligning the compared test sequence to the refence sequence. Sequence identity is most preferably assessed by alignment with the BLAST version 2.1 program advanced search (parameters as above). BLAST is a series of programs that are available online at blast.ncbi.nlm.nih.gov/. The BLAST search may be set to de-fault parameters (i.e. Matrix BLOSUM62; Gap existence cost 11; Per residue gap cost 1; Lambda ratio 0.85 default). References to BLAST searches are: Altschul et al., J. Mol. Biol. (1990) 215: 403-410; Gish & States, Nature Genet. (1993) 3: 266-272; Madden et al., Meth. Enzymol. (1996) 266: 131-141; Altschul et al. Nucleic Acids Res. (1997) 25: 3389-3402. Identical amino acids in the alignment are counted and the number of identical amino acids over the length of the reference sequence give the ratio of identical amino acids, preferably expressed in %.

Preferably, CLEC4G or the CRD of CLEC4G have a similar length as human CLEC4G (SEQ ID NO: 2) or the CRD of human CLEC4G (aa 165-288 of SEQ ID NO: 2), respectively. Preferably, there are at most 80, preferably at most 50, amino acid deletions or additions in the sequence of CLEC4G or the CRD of CLEC4G in comparison to human CLEC4G or the CRD of CLEC4G. Amino acid additions at the end, e.g. as in a fusion protein are irrespectively possible. Such fusion proteins include preferred embodiments as described further below, such as fusion with immunoglobulin domains. As such, this number refers to amino acids within the reference sequence while additions at the end of the reference sequence are not counted. Some deletions may be possible. Preferably, in the inventive CRD, about 1, 2, 3, 4, 5, 6 to 10, 11 to 25, amino acids of the CRD of amino acids 165-288 of SEQ ID NO: 2 are modified or deleted. Also here, this number refers to amino acids within the reference sequence while additions at the end of the reference sequence are not counted.

Non-identities in the amino acids are preferably conservative changes or substantially conservative changes. Accordingly, the invention includes polypeptides having conservative changes or substitutions in amino acid sequences. Conservative amino acid substitutions insert one or more amino acids, which have similar chemical properties as the replaced amino acids. The invention includes sequences where conservative amino acid substitutions are made that do not abrogate binding to Spike glycoprotein of SARS-CoV-2.

"Conservative amino acid substitutions" are those substitutions that are predicted to interfere least with the properties of the reference polypeptide. In other words, conservative amino acid substitutions substantially conserve the structure and the function of the reference protein. The following Table provides a list of exemplary conservative amino acid substitutions:

| Original Residue | Conservative Substitution |
|---|---|
| Ala | Gly, Ser |
| Arg | His, Lys |
| Asn | Asp, Gln, His |
| Asp | Asn, Glu |
| Cys | Ala, S er |
| Gln | Asn, Glu, His |
| Glu | Asp, Gln, His |
| Gly | Ala |
| His | Asn, Arg, Gln, Glu |
| Ile | Leu, Val |
| Leu | Ile, Val |
| Lys | Arg, Gln, Glu |
| Met | Leu, Ile |
| Phe | His, Met, Leu, Trp, Tyr |
| Ser | Cys, Thr |
| Thr | Ser, Val |
| Trp | Phe, Tyr |
| Tyr | His, Phe, Trp |
| Val | Ile, Leu, Thr |

Conservative amino acid substitutions generally maintain one or more of: (a) the structure of the polypeptide backbone in the area of the substitution, for example, as a beta sheet or alpha helical conformation, (b) the charge or hydrophobicity of the molecule at the site of the substitution, and/or (c) the bulk of the side chain. Preferably, at least 50% of the non-identical amino acids in the CRD to the CRD of human CLEC4G are conservative amino acid substitutions.

Preferably, the polypeptide comprises CLEC4G or only an extracellular domain of CLEC4G. Preferably, the polypeptide lacks the cytoplasmic domain of CLEC4G and/or the transmembrane domain of CLEC4G, in particular preferred lacks any cytoplasmic domain and/or any transmembrane domain. In further preferred embodiments, as alternative or combinable with the lack of a cytoplasmic domain and/or of a transmembrane domain, only a part of a CLEC4G protein is used. Accordingly, the polypeptide may comprise 50 to 250 amino acids in length of CLEC4G. In preferred embodiments, the polypeptide may comprise 70 to 200, even more preferred 80 to 180 or 100 to 150, amino acids in length of CLEC4G.

Preferably, the polypeptide comprises a homolog of the CRD of human CLEG4G from any mammal, such as mouse, rat, hamster, cow, cat, non-human primate, monkey, pig, horse, dog, etc.

In preferred embodiments the polypeptide comprises an immunoglobulin domain. More than one immunoglobulin domain is possible. One or more immunoglobulin domains can be added e.g. as in a fusion protein. A fusion protein may comprise a linker, such as short peptide connecting different parts of the fusion protein. E.g. the linker may be located between a part comprising the carbohydrate recognition domain of CLEC4G, and a part comprising the immunoglobulin domain(s). Preferably, the immunoglobulin domain comprises or consists of antibody CH1, CH2 or CH3 domain, or combinations thereof.

The immunoglobulin domain may be of a human immunoglobulin domain. It may be of an IgG, such as an IgG1, IgG2, IgG3, IgG4, preferably IgG1, IgA, such as IgA1 or IgA2, IgGD, IgE, IgM or combinations thereof. Preferably, the polypeptide comprises an antibody Fc fragment. An Fc fusion is preferably to an Fc of IgG, IgM, IgD, or IgA or a part thereof, such as a CH1, CH2 or CH3 domain, or FcRn. A CH3 domain is preferred. It may or may not include the C-terminus of the Fc part. IG is preferably human IgG1, IgG2, and IgG4.

In further preferred embodiments the polypeptide is a dimer.

Preferably, the CLEC4G is human CLEC4G and the patient is a human. Any part of human CLEC4G, in particular the CRD thereof, as described above may be used.

As used herein, the term "subject" may be used interchangeably with the term "patient" or "individual" and may include an "animal" and in particular a "mammal", that can be treated according to the invention. Mammalian subjects may include humans and non-human primates, domestic animals, farm animals, and companion animals such as dogs, cats, guinea pigs, rabbits, rats, mice, horses, cattle, cows, and the like. Preferably, the patient to be treated by the inventive method and uses of the invention is a human, preferably a human adult of the age of 18 years or more.

The inventive polypeptide is used to treat an infection with a coronavirus expressing a SARS-CoV-2 Spike protein. This includes treatment of COVID-19 and any infections with various strains and variants of SARS-CoV-2 or with any coronavirus that expresses a SARS-CoV-2 Spike protein, including variations thereof.

A main variant of the SARS-CoV-2 Spike protein is provided in UniProtKB database entry P0DTC2, entry version of 2 December 2020, provided as SEQ ID NO: 1 herein.

Preferably, the SARS-CoV-2 Spike protein has a sequence identity of at least 85%, preferably at least 90%, more preferred at least 95%, to SEQ ID NO: 1. Sequence identity is determined as discussed above.

Preferably, the infection is an infection with SARS-CoV-2. The infection may be of any SARS-CoV-2 variant or strain, including 20A.EU1, 20A.EU2, B.1.1.7 (also known as 20I/501Y.V1), B.1.351 (also known as 20H/501Y.V2), P.1 (also known as 20J/501Y.V3), 20B/S.484K, as well as variants or strains with mutations or deletions in the Spike protein, including S:S13I, S:W152C, S:A222V, S:K417N, S:N439K, S:D614G, S:A222V, S:S477N, S:N501, especially S:N501Y, S:N501T, S:N501S, S:E484, especially S:E484K, S:N453F, S:S98F, S:L452R, S:D80Y, S:A626S, S:I692V, S:V1122L, S:M1229I, S:A570D, S:D614G, S:P681H, S:T716I, S:S982A, S:D1118H, and deletion variant with deletion S:H69-, S:V70-, S:69/70-, S:144-, or variants with mutations at other loci, such as ORF10:V30L, N: S186Y, N: D377Y, N:P199L, N:A220V, N:M234I, N:A376T, ORF1b:A176S, ORF1b:V767L, ORF1b:K1141R, ORF1b:E1184D, ORF1a:I2501T, ORF3a:Q38R, ORF3a:G172R, ORF3a:V202L, ORF1a:I4205V, ORF1b:D1183Y, ORF1a:T945I, ORF1a:T1567I, ORF1a:Q3346K, ORF1a:V3475F, ORF1a:M3862I, ORF1b:P255T, ORF7a:R80I, or combinations thereof, such as in B.1.1.7, e.g. a combination of S:deletion 69-70, S:deletion 144, S:N501Y, S:A570D, S:D614G, S:P681H, S:T716I, S:S982A, S:D1118H.

In accordance with the invention, a pharmaceutical composition or medicine comprising the polypeptide can be provided. The pharmaceutical composition may be in a container, such as a vial, flask, syringe, bag, and/or in kit. Such compositions may be pharmaceutically acceptable salts themselves, with additional buffers, tonicity components or pharmaceutically acceptable carriers. Pharmaceutical carrier substances serve to improve the compatibility of the composition and provide better solubility as well as better bioavailability of the active ingredients. Examples are emulsifiers, thickeners, redox components, starches, alcoholic solutions, polyethylene glycol and lipids. Selection of a suitable pharmaceutical carrier is highly dependent on the administration route. For oral administration, liquid or solid carriers may be used; for injections, liquid final compositions are required.

Preferably, the polypeptide is provided in a composition comprising buffers or tonic substances. The buffer can adjust the pH of the medicine to the physiological conditions and further, can reduce or buffer variations in pH. An example is a phosphate buffer. Tonic substances can adjust the osmolarity and may include ionic substances, such as inorganic salts, for example NaCl or KCl, or non-ionic substances such as glycerin or carbohydrates.

Preferably, the composition for use in accordance with the invention is suitably prepared for systemic, topical, oral or intranasal administration or as an inhaled preparation. Such administration routes are preferred embodiments of the inventive methods. These forms of administration for the composition of the present invention allow fast, uncomplicated take-up. When the polypeptide is intended for oral administration, it is preferably provided in a formulation which is resistant to stomach acid or it is encapsulated. For oral administration, solid or liquid medicines can be taken directly or dissolved or diluted, for example. The pharmaceutical composition or kit for use in accordance with the invention is preferably produced for intravenous, intra-arterial, intramuscular, intravascular, intraperitoneal or subcutaneous administration. Injections or transfusions, for example, are suitable for this purpose. Administration directly into the bloodstream has the advantage that the active ingredient of the medicine can be distributed through the entire body and the target tissue, such as lungs, heart, kidney, intestine or liver, is reached quickly.

The composition may be pharmaceutically acceptable. The term "pharmaceutically acceptable" indicates that the designated carrier, vehicle, diluent, excipient(s), and/or salt is generally chemically and/or physically compatible with the other ingredients comprising the formulation, and physiologically compatible with the recipient thereof. In some embodiments, compounds, materials, carriers, compositions, and/or dosage forms that are pharmaceutically acceptable refer to those approved by a regulatory agency (such as U.S. Food and Drug Administration, National Medicine or European Medicines Agency) or listed in generally recognized pharmacopoeia (such as U.S. Pharmacopoeia, China Pharmacopoeia or European Pharmacopoeia) for use in animals, and more particularly in humans.

Pharmaceutical acceptable carriers for use in the pharmaceutical compositions disclosed herein may include, for example, pharmaceutically acceptable liquid, gel, or solid carriers, aqueous vehicles, non-aqueous vehicles, antimicrobial agents, isotonic agents, buffers, tonicity-adjusting agents, antioxidants, anesthetics, suspending/dispending agents, sequestering or chelating agents, diluents, adjuvants, excipients, or non-toxic auxiliary substances, other components known in the art, or various combinations thereof. Suitable carriers and auxiliary components may include, for example, fillers, binders, disintegrants, buffers, preservatives, lubricants, flavorings, thickeners, coloring agents, emulsifiers or stabilizers such as sugars and cyclodextrins. In some embodiments, the suitable buffers may include, for example, a phosphate buffer or a MES (2-(N-morpholino)ethane sulfonic acid) buffer.

To further illustrate, pharmaceutical acceptable carriers may include, for example, aqueous vehicles such as sodium chloride injection, Ringer's injection, isotonic dextrose injection, sterile water injection, or dextrose and lactated Ringer's injection, nonaqueous vehicles such as fixed oils of vegetable origin, cottonseed oil, corn oil, sesame oil, or peanut oil, antimicrobial agents at bacteriostatic or fungistatic concentrations, isotonic agents such as sodium chloride or dextrose, buffers such as phosphate or citrate buffers or MES (2-(N-morpholino)ethane sulfonic acid) buffers, antioxidants such as sodium bisulfate, local anesthetics such as procaine hydrochloride, suspending and dispersing agents such as sodium carboxymethylcelluose, hydroxypropyl methylcellulose, or polyvinylpyrrolidone, emulsifying agents such as Polysorbate 80 (TWEEN-80), sequestering or chelating agents such as EDTA (ethylenediaminetetraacetic acid) or EGTA (ethylene glycol tetraacetic acid), ethyl alcohol, polyethylene glycol, propylene glycol, sodium hydroxide, hydrochloric acid, citric acid, or lactic acid. Antimicrobial agents utilized as carriers may be added to pharmaceutical compositions in multiple-dose containers that include phenols or cresols, mercurials, benzyl alcohol, chlorobutanol, methyl and propyl p-hydroxybenzoic acid esters, thimerosal, benzalkonium chloride and benzethonium chloride. Suitable excipients may include, for example, water, saline, dextrose, glycerol, or ethanol. Suitable non-toxic auxiliary substances may include, for example, wetting or emulsifying agents, pH buffering agents, stabilizers, solubility enhancers, or agents such as sodium acetate, sorbitan monolaurate, triethanolamine oleate, or cyclodextrin.

The pharmaceutical compositions with the polypeptide can be a liquid solution, suspension, emulsion, pill, capsule, tablet, sustained release formulation, or powder. Oral formulations can include standard carriers such as pharmaceutical grades of mannitol, lactose, starch, magnesium stearate, polyvinyl pyrollidone, sodium saccharine, cellulose, magnesium carbonate, etc.

The form of pharmaceutical compositions depends on a number of criteria, including, but not limited to, route of administration, extent of disease, or dose to be administered. The pharmaceutical compositions can be formulated for intravenous, oral, nasal, rectal, percutaneous, or intramuscular administration. For example, dosage forms for intravenous administration, may be formulated as lyophilized powder or fluid formulation; dosage forms for nasal administration may conveniently be formulated as aerosols, solutions, drops, gels or dry powders. In accordance to the desired route of administration, the pharmaceutical compositions can be formulated in the form of tablets, capsule, pill, dragee, powder, granule, sachets, cachets, lozenges, suspensions, emulsions, solutions, syrups, aerosols (as a solid or in a liquid medium), spray, inhalant, or suppository.

In some embodiments, the pharmaceutical compositions are formulated into an injectable composition. The injectable pharmaceutical compositions may be prepared in any conventional form, such as for example liquid solution, suspension, emulsion, or solid forms suitable for generating liquid solution, suspension, or emulsion. Preparations for injection may include sterile and/or non-pyretic solutions ready for injection, sterile dry soluble products, such as lyophilized powders, ready to be combined with a solvent just prior to use, including hypodermic tablets, sterile suspensions ready for injection, sterile dry insoluble products ready to be combined with a vehicle just prior to use, and sterile and/or non-pyretic emulsions. The solutions may be either aqueous or nonaqueous. Aqueous is preferred.

In some embodiments, unit-dose i.v. or parenteral preparations are packaged in an ampoule, a vial, bag or a syringe with a needle. All preparations for parenteral administration should be sterile and not pyretic, as is known and practiced in the art.

Depending on the route of administration, the polypeptide can be coated with or disposed in a selected material to protect it from natural conditions which may detrimentally affect its ability to perform its intended function. It may be administered alone, or in conjunction with a pharmaceutically acceptable carrier.

Preferably, the polypeptide or nucleic acid is administered by inhalation, intravenous, intraarterial, intramuscular, intravascular, intraperitoneal, sub-cutaneous or oral administration.

Also disclosed is a polypeptide or nucleic acid for use in a method of treating a coronavirus infection comprising administering a polypeptide comprising a carbohydrate recognition domain of CD209c or a nucleic acid encoding said polypeptide to a patient suffering from an infection with a coronavirus expressing a SARS-CoV-2 Spike protein. The entire disclosure herein as disclosed for CLEC4G also equivalently applies to CD209c.

The invention further provides a library of at least 10 different polypeptides, each polypeptide comprising a different carbohydrate recognition domain of a lectin and an immunoglobulin domain.

A library is a collection of polypeptides of the invention. The polypeptides of the library may be provided in separate containers, such as flasks, wells, cavities, vials and the like. The individual containers may be combined in a kit-in-parts, wherein the kit contains these containers. A kit may comprise a packaging, such as further containers including the individual containers of the polypeptides. In other embodiments, the polypeptides may be provided in a mixture. In such a mixture, the polypeptides may remain identifiable through their amino acid sequence or through the use of different labels, such as nucleic acid labels.

Carbohydrate recognition domains (CRDs) of lectins are domains for binding carbohydrates as known in the art. An example CRD of CLEC4G has been described above. They are usually found on an extracellular part of the lectins. Preferably, such extracellular parts of the domains themselves of the lectins may be provided. Preferably, the polypeptides lack a lectin membrane domain and or lack a lectin cytosolic domain. The carbohydrate recognition domains (CRDs) of lectins may be homologous to the CRD of CLEC4G and/or may correspond to amino acids 165-288 of SEQ ID NO: 2. The CRDs are usually short sequences. Preferably, the polypeptide comprises 100 to 250 amino acids of a CRD or a lectin or of CLEC4G. In preferred embodiments, the polypeptide may comprise 70 to 200, even more preferred 80 to 180 or 100 to 150, amino acids of a CRD or a lectin or of CLEC4G. Further parts are possible, especially connected to an end of the CRD containing part of the polypeptide, such as in a fusion protein.

In particular, the polypeptides contain an immunoglobulin domain, as described above, such as an Fc part of IgG, IgM, IgD, or IgA or a part thereof, such as a CH1, CH2 or CH3 domain, or FcRn.

Preferably, the lectins are selected from C-type lectins, galectins and siglecs. These types of lectins are a preferred selection of with potential for the inventive uses, most prominently screening for therapeutic uses.

In particular preferred embodiments, at least 10, preferably at least 20, at least 30, at least 40, at least 50, CRDs are selected from the different lectins of table 1. The lectins of table 1 (lectins given in the right-most column) are particular suitable for the inventive purposes, such as screening for therapeutic potential.

Also provided is a set of nucleic acids encoding the polypeptides of a library of the invention. The nucleic acids can be used to express the inventive polypeptides.

Further provided is a method of identifying or screening a lectin candidate that binds to a carbohydrate-containing target of interest comprising contacting the target with the polypeptides of a library of the invention, and detecting polypeptides bound to said target.

A carbohydrate-containing target of interest may be a carbohydrate itself or a carbohydrate fused to a protein, fatty acid or lipid, such as a glycoprotein or a carbohydrate on a cell membrane. The carbohydrate-containing target may be a bacterial protein, such as virulence factor, or a cell binding protein, e.g. utilised by a pathogenic cell or virion to bind to cells.

In preferred embodiments, the carbohydrate-containing target is a viral protein, a bacterial protein, a fungal protein or a cancer-associated protein. A cancer-associated protein may be a protein that is expressed or overexpressed or expressed in altered form and contributes to a cancer cells amplification, immortality, tissue invasion or immune evasion.

Preferably, the method further comprises immobilization of the target and detecting polypeptides that are immobilized through binding the immobilized target; and/or wherein the polypeptides are labelled by binding a labelled immunoglobulin-binding moiety to the polypeptides' immunoglobulin domain. An example of such a method is an immune assay, such as an ELISA.

Throughout the present disclosure, the articles "a", "an", and "the" are used herein to refer to one or to more than one (i.e., to at least one) of the grammatical object of the article.

As used herein, words of approximation such as, without limitation, "about", "substantial" or "substantially" refer to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skill in the art recognize the modified feature as still having the required characteristics and capabilities of the unmodified feature. In general, but subject to the preceding discussion, a numerical value herein that is modified by a word of approximation such as "about" may vary from the stated value by e.g. ±10%.

As used herein, the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The "comprising" expressions when used on an element in combination with a numerical range of a certain value of that element means that the element is limited to that range while "comprising" still relates to the optional presence of other elements. E.g. the element with a range may be subject to an implicit proviso excluding the presence of that element in an amount outside of that range. As used herein, the phrase "consisting essentially of" requires the specified integer(s) or steps as well as those that do not materially affect the character or function of the claimed invention. As used herein, the closed term "consisting" is used to indicate the presence of the recited elements only.

The present invention is further described by the following figures and examples, without necessarily being limited to these embodiments of the invention.

### Figures

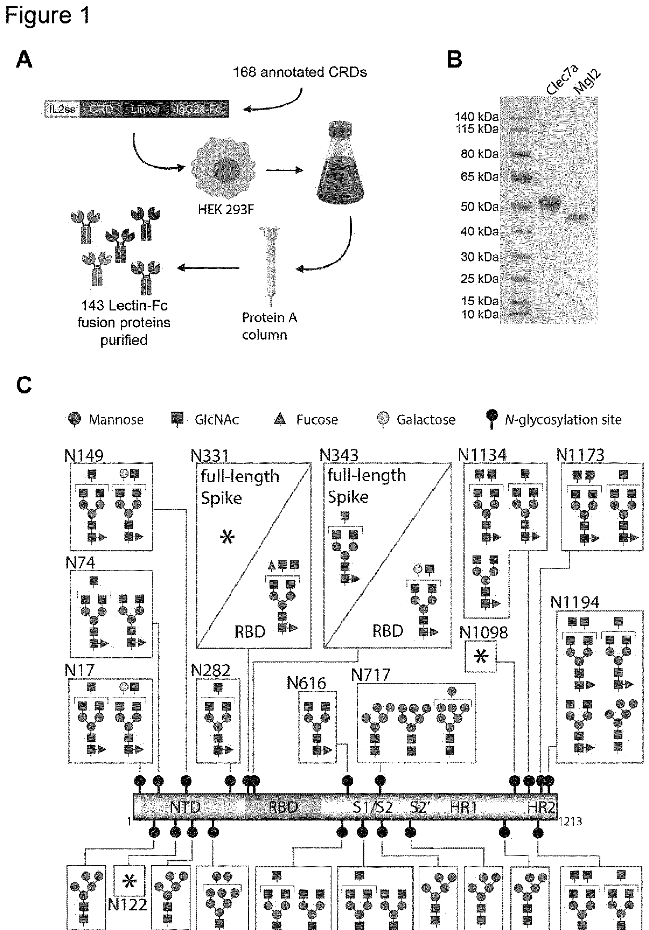

**Figure 1****. Lectin library and SARS-CoV-2 Spike and RBD glycosylation. (A)** Schematic overview of cloning, expression and purification of 143 carbohydrate recognition domain (CRD) - mouse IgG2a-Fc fusion proteins, from 168 annotated murine CRD containing proteins. The constructs were expressed in HEK293F cells and secreted Fc-fusion proteins were purified using protein A columns. See table 1 for full list of expressed CRDs. **(B)** Exemplified SDS-PAGE of purified Clec7a and Mgl2 stained with Coomassie blue. **(C)** Glycosylation map of the SARS-CoV-2 Spike and RBD. The most prominent glycan structures are represented for each site, with at least 15% relative abundance. * marks highly variable glycosylation sites in which no single glycan structure accounted for >15% relative abundance. The different monosaccharides are indicated using standardized nomenclature. NTD, n-terminal domain; RBD, receptor binding domain; S1/S2 and S2', proteolytic cleavage sites; HR1 and HR2, α-helical heptad repeat domains 1 and 2; GlcNAc, N-acetylglucosamine.

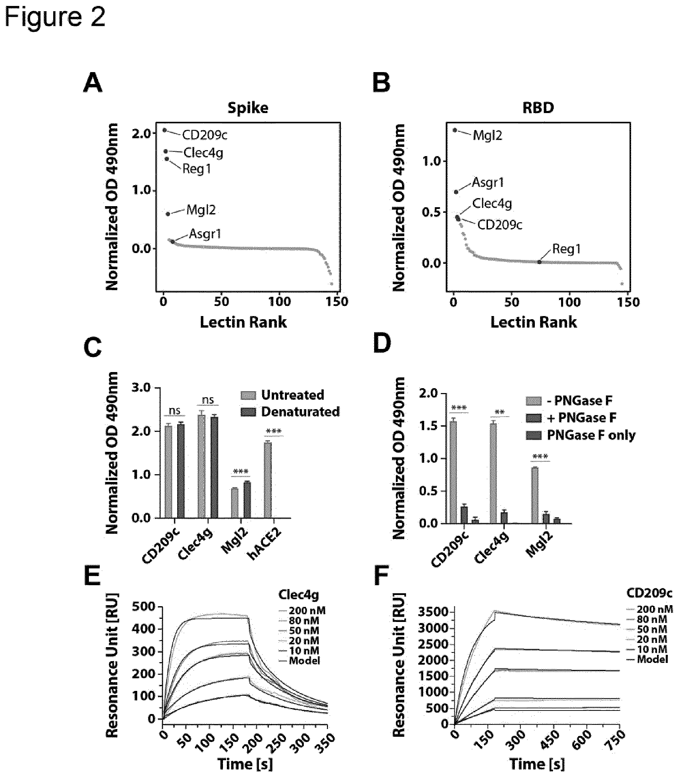

**Figure 2****. Identification of lectins that bind to Spike and RBD of SARS-CoV-2. (A)** and **(B)** ELISA screen of the lectin-Fc library against full-length trimeric SARS-CoV-2 Spike (A) or monomeric RBD (B). Results are shown as mean OD values of 2 replicates normalized against a BSA control and ranked by value. Lectin-Fc fusion proteins with a normalized OD > 0.5 in either (A) or (B) are indicated in both panels. See table 2 for primary ELISA data. **(C)** Lectin-Fc and human ACE2-mIgG1 Fc-fusion protein (hACE2) binding to untreated or heat-denatured full-length SARS-CoV-2 Spike by ELISA. hACE2-mIgG1 was used as control for complete denaturation of Spike protein. Results are shown as mean OD values ± SD normalized to the BSA control (N=3). **(D)** Lectin-Fc binding to full-length SARS-CoV-2 Spike with or without de-N-glycosylation by PNGase F. "PNGase F only" denotes wells that were not coated with the Spike protein. Results are shown as mean OD values ± SD normalized to BSA controls (N=3). **(E)** and **(F)** Surface plasmon resonance (SPR) analysis with immobilized full-length trimeric Spike, probed with various concentrations of Clec4g-Fc (E) and CD209c-Fc (F). See Table 3 for kinetics values. (C) t-test with Holm-Sidak correction for multiple comparisons. (D) One-way ANOVA with Tukey's multiple comparisons; *P<0.05; **P<0.01; ***P<0.001; ns: not significant.

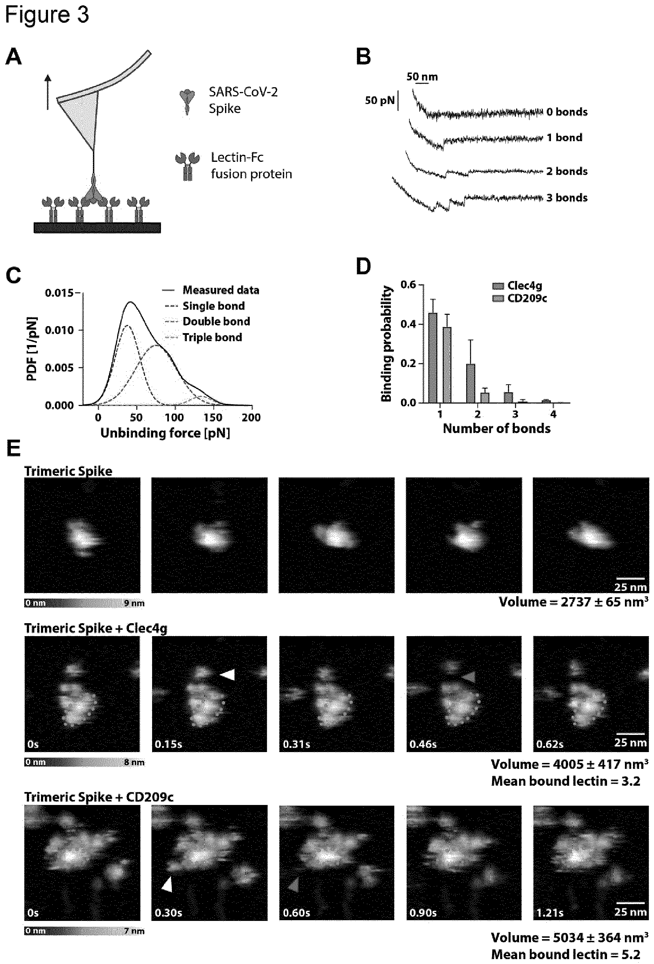

**Figure 3****. Single molecule, real time imaging of lectin-Spike binding. (A)** Schematic overview of single molecule force spectroscopy (SMFS) experiments using full-length trimeric Spike coupled to an atomic force microscopy (AFM) cantilever tip and surface coated murine Clec4g-Fc or CD209c-Fc. Arrow indicates pulling of cantilever. **(B)** Representative force traces showing sequential bond ruptures in the SMFS experiments. Measured forces are shown in pico-Newtons (pN). **(C)** Experimental probability density function (PDF) of unbinding forces (in pN) determined by SMFS (black line, measured data). The three distinct maxima fitted by a multi-Gaussian function reveal rupture of a single bond (blue dotted line), or simultaneous rupture of 2 (red dotted line) and 3 (green dotted line) bonds, respectively. **(D)** SMFS-determined binding probability for the binding of trimeric Spike to Clec4g and CD209c. Data are shown as mean binding probability ± SD of single, double, triple or quadruple bonds (N=2). **(E)** High speed AFM of single trimeric Spike visualizing the real-time interaction dynamics with lectins. Top panel shows 5 frames of trimeric Spike alone imaged on mica. Middle and bottom panels show 5 sequential frames of trimeric Spike/Clec4g and trimeric Spike/CD209c complexes, acquired at a rate of 153.6 and 303 ms/frame, respectively. Association and dissociation events between lectin and Spike are indicated by white and red arrows, respectively. The blue dotted ellipses display the core of the complexes showing low conformational mobility. Color schemes indicate height of the molecules in nanometers (nm). Volumes of single trimeric Spike, trimeric Spike/Clec4g and trimeric Spike/CD209c complexes are indicated, as well as numbers of lectins bound to trimeric Spike, averaged over the experimental recording period.

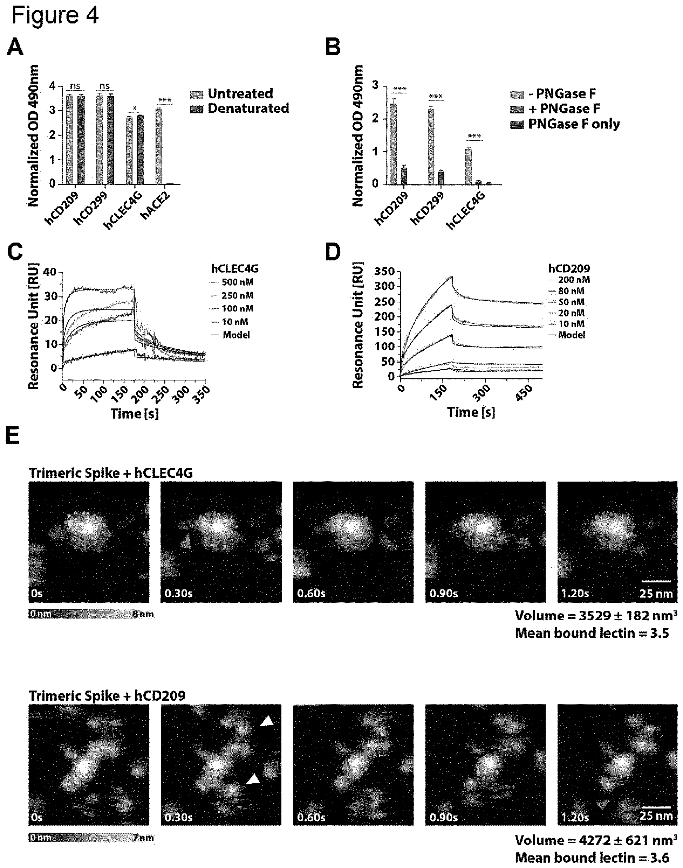

**Figure 4****. Characterization of human lectin-Spike interactions. (A)** ELISA analyses of human lectin-hIgG1 Fc-fusion protein (hCLEC4g, hCD209, hCD299) binding to untreated or heat-denatured full-length SARS-CoV-2 Spike. A human ACE2-hIgG1-Fc fusion protein (hACE2) was used to control for the complete denaturation of Spike. Results are shown as mean OD values ± SD normalized to a BSA control (N=3). **(B)** hCLEC4g, hCD209, and hCD299 binding to full-length SARS-CoV-2 Spike with or without de-N-glycosylation by PNGase F. "PNGase F only" denotes wells that were not coated with the Spike protein. Results are shown as mean OD values ± SD normalized against the BSA control (N=3). **(C)** and **(D)** SPR analysis with immobilized full-length trimeric Spike, probed with various concentrations of hCLEC4G (C) and hCD209 (D). See Table 3 for kinetics values. **(E)** High speed AFM of single trimeric Spike visualizing the real-time interaction dynamics with lectins. Top and bottom panels show 5 sequential frames of trimeric Spike/hCLEC4G and trimeric Spike/hCD209 complexes, acquired at a rate of 303 ms/frame. Association and dissociation events between hCLEC4G or hCD209 and Spike are indicated by white and red arrows, respectively. The blue dotted ellipses display the core of the complexes showing low conformational mobility. Color schemes indicate height of the molecules in nanometers (nm). Volumes of single trimeric Spike and trimeric Spike/Clec4g and trimeric Spike/CD209c complexes are indicated, as well as numbers of lectins bound to trimeric Spike, averaged over the experimental recording period. (A) t-test with Holm-Sidak correction for multiple comparisons. (B) One-way ANOVA with Tukey's multiple comparisons; *P<0.05; ***P<0.001; ns: not significant.

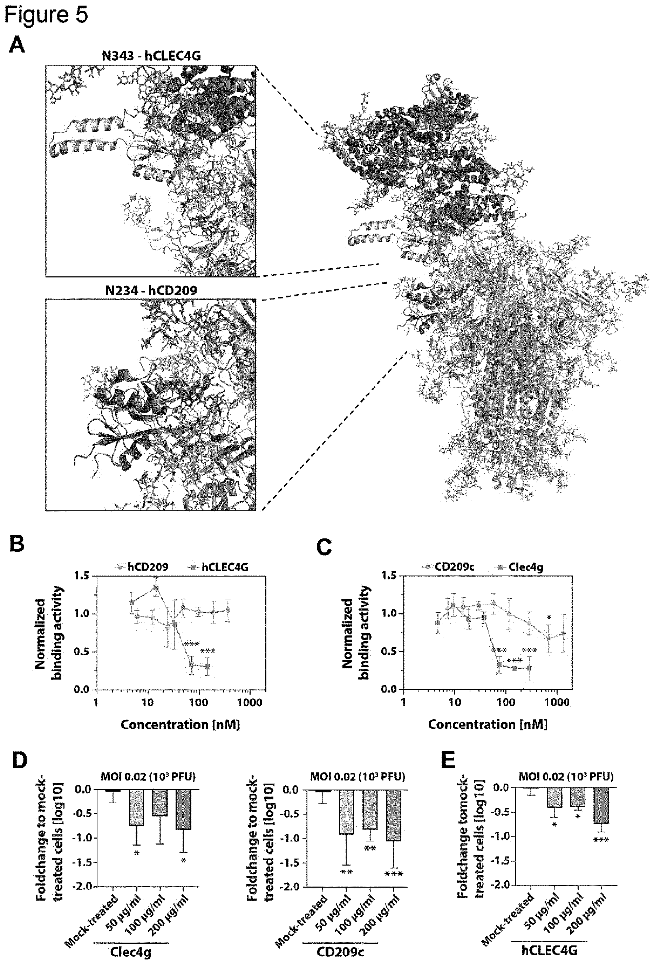

**Figure 5****. Structural modelling and functional determination of lectin-Spike binding in SARS-CoV-2 infections. (A)** 3D structural modelling of glycosylated trimeric Spike (green with glycans in yellow) interacting with glycosylated human ACE2 (purple with glycans in salmon). The CRD of hCLEC4G (cyan with Ca²⁺ in orange) was modelled onto Spike monomer 3 glycan site N343 (complex type glycan with terminal GlcNAc in purple-blue) and the CRD of hCD209 (dark blue with Ca²⁺ in orange) was modelled onto the Spike monomer 3 glycan site N234 (Oligomannose structure Man9 in red). Structural superposition of CLEC4G and ACE2 highlights sterical incompatibility. **(B)** and **(C)** Binding activity of the full-length trimeric Spike coupled to an AFM cantilever tip to the surface of Vero E6 cells in the presence of (B) hCLEC4G and hCD209 or (C) mouse Clec4g or murine CD209c, probed at the indicated concentrations in SMFS experiments. A definite decrease in binding was observed at concentrations of 37 - 75 nM for Clec4g and 33 - 71 nM for hCLEC4G. Data are normalized to the untreated control and shown as mean ± SD (N=4). **(D)** Infectivity of mouse Clec4g or CD209c and **(E)** hCLEC4G pre-treated SARS-CoV-2 virus in Vero E6 cells. Viral RNA was measured with qRT-PCR 15 hours after infection with 0.02 MOI (10³ PFUs) of SARS-CoV-2. Data represent two pooled experiments for mouse lectins (N=5-6) and one experiment for hCLEC4g (N=3) and are presented as fold changes of viral loads over mock-treated controls (mean log10 values ± SD). (B) - (E) ANOVA with Dunett's multiple comparisons test with the mock-treated group; *P<0.05; **P<0.01; ***P<0.001.

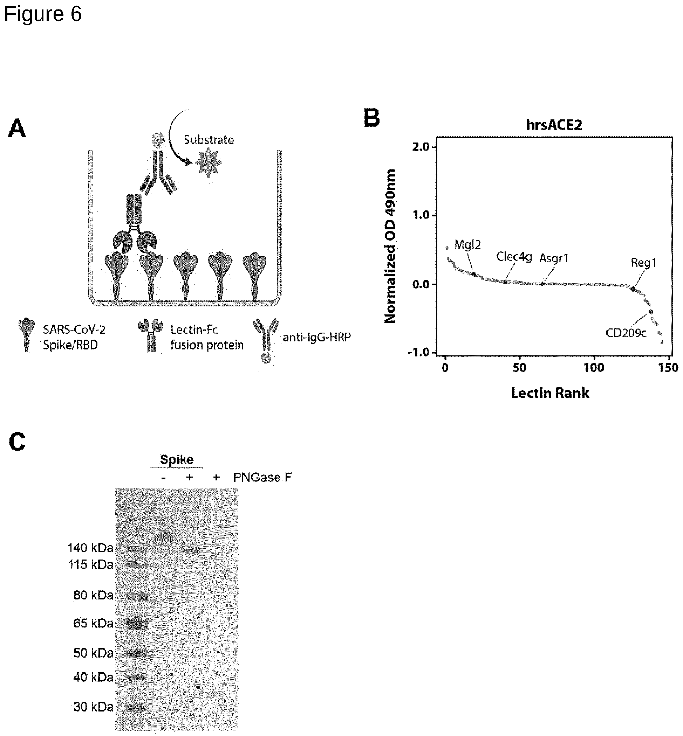

**Figure 6****. ELISA assays to detect lectin binding. (A)** Schematic representation of the ELISA protocol, consisting of coating with trimeric full-length Spike or the monomeric receptor binding domain (RBD) followed by sequential incubation with lectin-Fc fusion proteins and secondary anti-IgG-HRP antibodies. The binding of lectin-Fc fusion proteins was quantified by peroxidase-dependent substrate conversion, measured by optical density (OD) at 490nm and normalized against a BSA control. **(B)** ELISA screen of the lectin-Fc library against human recombinant soluble ACE2 (hrsACE2). Results are shown as mean OD values of 2 replicates normalized against a BSA control and ranked by value. **(C)** SDS-Page of full-length Spike de-*N*-glycosylated with PNGase F and stained with Coomassie blue. A PNGase F control was added to display the size of the PNGase F protein.

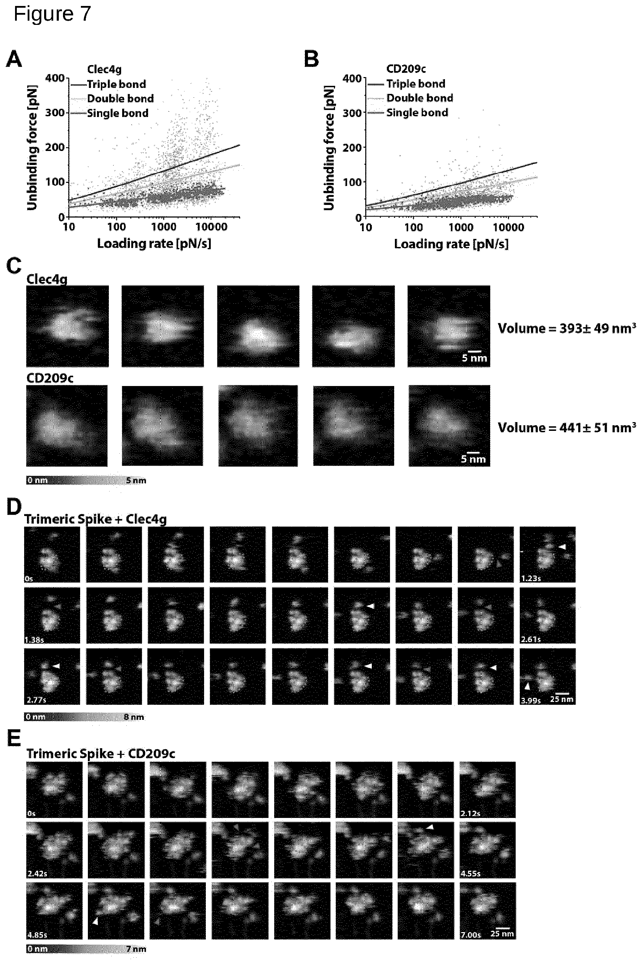

**Figure 7****. Single molecule atomic force microscopy of a single trimeric Spike binding to murine Clec4g or CD209c. (A)** and **(B)** Unbinding forces versus loading rates for trimeric Spike dissociating from (A) Clec4g-Fc or (B) CD209c-Fc. Unbinding forces were determined from the magnitude of the vertical jumps measured during pulling of the cantilever (Fig. 3B) and individually plotted versus the respective force loading rates (equal to the pulling speed times effective spring constant) to decipher the dissociation dynamics (Table 3). A well-defined single-bond behavior of a unique monovalent bond was found (red dots) that, in line with Evans's single energy barrier model, yielded a linear rise of the unbinding force with respect to a logarithmically increasing loading rates for both (A) Clec4g and (B) CD209c. Double (green) and triple (blue) bond behaviors were calculated according to the Markov binding model using parameters derived from the single barrier model. Unbinding force values scattered between single and triple bond strengths, indicating that interactions with various glycosylation sites with different binding strengths. pN=picoNewton, pN/s = picoNewton per second. **(C) - (E)** High speed AFM of a single trimeric Spike visualizing the real-time interaction dynamics with lectins. (C) 5 frames of Clec4g or CD209c alone imaged on mica. (D) Sequential movie frames of trimeric Spike/Clec4g complexes, acquired at a rate of 153.6 ms/frame, corresponding to Fig. 3C. (E) Sequential movie frames of trimeric Spike/CD209c complexes, acquired at 303 ms/frame, corresponding to Fig. 3C. White arrows point to lectins associating with the Spike trimer body. Red arrows indicate dissociation of lectins from the Spike trimer, highlighting positions where the lectin was bound in the previous frame. Blue dotted ellipses display low mobility regions. Color schemes indicate height in nanometers (nm).

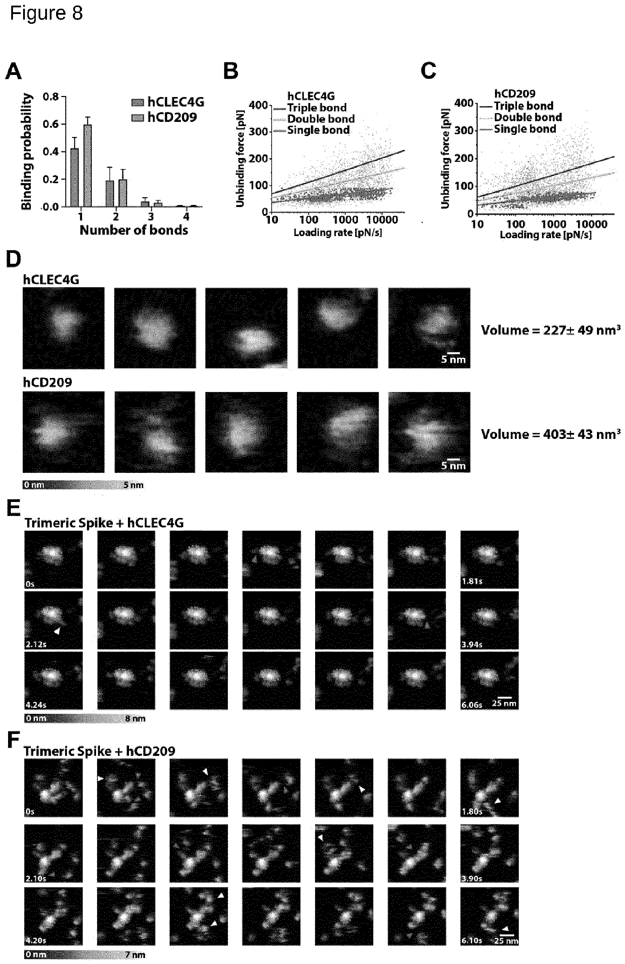

**Figure 8****. Single molecule atomic force microscopy of a single trimeric Spike binding to human CLEC4g or CD209. (A)** Single molecule force spectroscopy (SMFS) to determine the binding probability for trimeric Spike to mica coated hCLEC4G and hCD209. Data are shown as mean binding probabilities ± SD of single, double, triple or quadruple bonds (N=2). **(B)** and **(C)** Unbinding forces versus loading rates for a single trimeric Spike dissociating from (B) hCLEC4G or (C) hCD209. Unbinding forces were determined from the magnitude of the vertical jumps measured during pulling (Fig. 3B) and individually plotted vs. their force loading rates (equal to the pulling speed times effective spring constant) to assess the dissociation dynamics (Table 3). Single bond interactions (red dots) were fitted using the Bell-Evans single barrier model (red line). A well-defined single-bond behavior of a unique monovalent bond was found (red dots) that, in line with Evans's single energy barrier model, yielded a linear rise of the unbinding force with respect to a logarithmically increasing loading rate for both (B) hCLEC4g and (C) hCD209. Double (green) and triple (blue) bond behaviors were calculated according to the Markov binding model using parameters derived from the single barrier model. Unbinding force values scattered between single and triple bond strengths, indicating that they arise from multiple interactions with various glycosylation sites. pN=picoNewton, pN/s = picoNewton per second. **(D) - (F)** High speed AFM of a single trimeric Spike visualizing the real-time interaction dynamics with lectins. (D) 5 frames of hCLEC4g or hCD209 alone imaged on mica. (E) Sequential movie frames of trimeric Spike/hCLEC4g complexes, acquired at a rate of 303 ms/frame, corresponding to Fig. 4E. (F) Sequential movie frames of trimeric Spike/hCD209 complexes, acquired at 153.6 ms/frame, corresponding to Fig. 4E. White arrows point to lectins associating with the Spike trimer body. Red arrows indicate dissociation of lectins from the Spike trimer, highlighting positions where the lectin was bound in the previous frame. The blue dotted ellipses display low mobility regions. Color schemes indicate height in nanometers (nm).

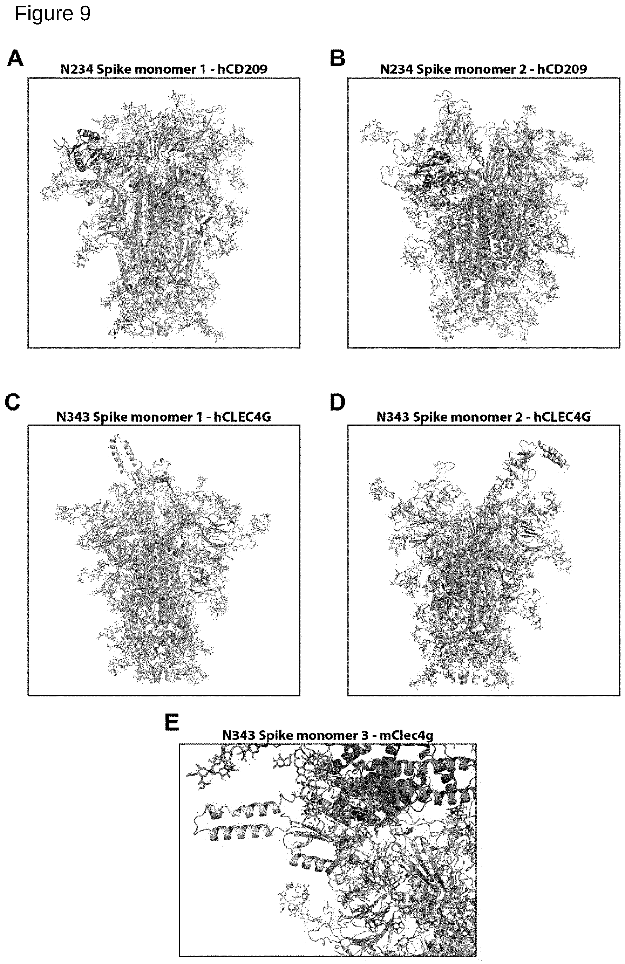

**Figure 9****. Structural modelling of lectin-Spike interactions. (A)** and **(B)** 3D structural modelling of glycosylated trimeric Spike (green with glycans in yellow) interacting with the CRD of hCD209 (dark blue with Ca²⁺ in orange). The model shows the (A) Spike monomer 1 and (B) Spike monomer 2 glycan site N234 (Oligo-mannose structure Man9 in red) bound to hCD209. **(C)** and **(D)** 3D structural modelling of glycosylated trimeric Spike (green with glycans in yellow) interacting with the CRD of hCLEC4g (cyan with Ca²⁺ in orange). The model shows Spike (A) monomer 1 and (B) monomer 2 glycan site N343 (complex type glycan with terminal GlcNAc in purple-blue) bound to hCLEC4g. **(E)** 3D structural modelling of glycosylated trimeric Spike (green with glycans in yellow) interacting with glycosylated human ACE2 (purple with glycans in salmon). The CRD of mClec4g (cyan with Ca²⁺ in orange) was modelled onto Spike monomer 3 glycan site N343 (complex type glycan with terminal GlcNAc in purple-blue). Structural superposition of mClec4g and ACE2 highlights steric incompatibility.

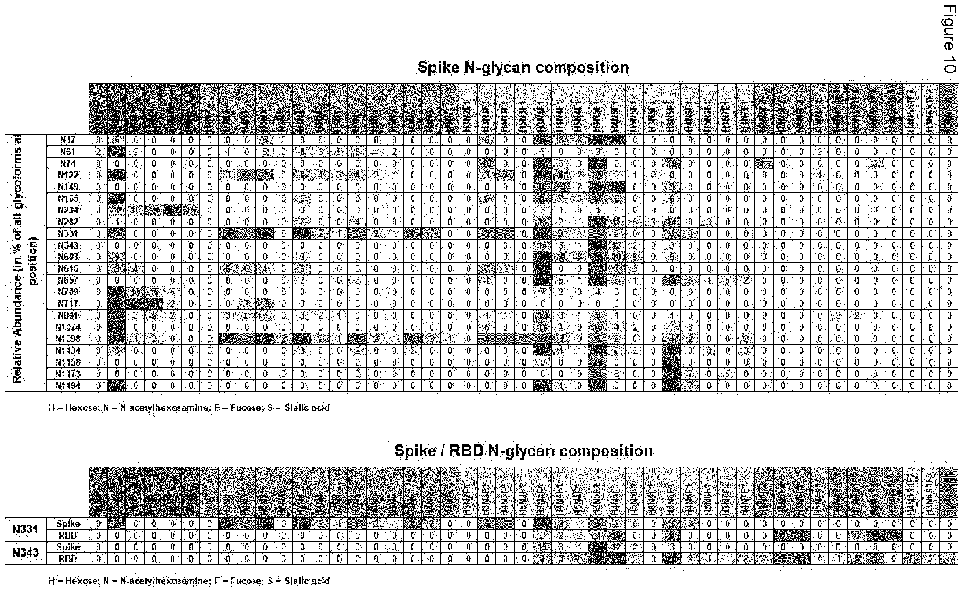

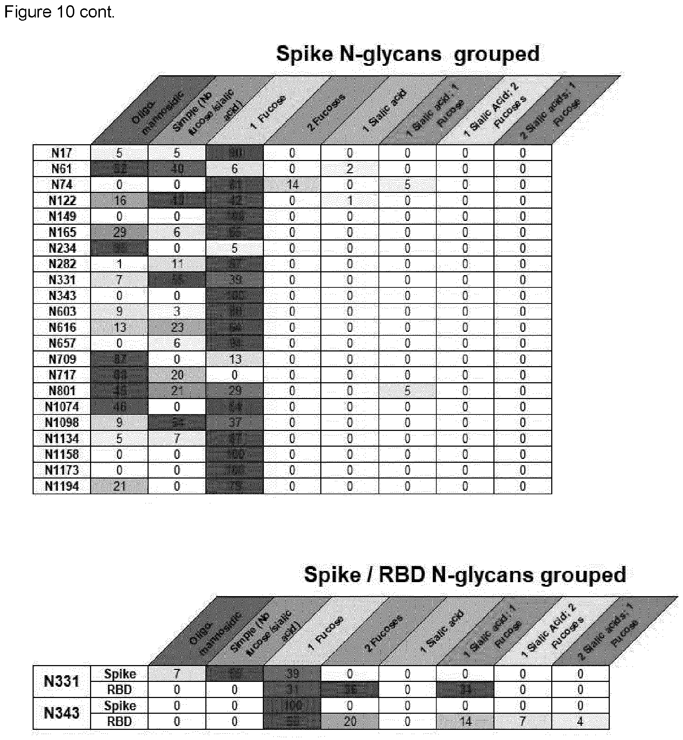

**Figure 10****. Glycosylation of SARS-CoV-2 Spike and RBD.** Relative abundance of all measured glycans in % of all glycans present at each position. Glycans are grouped in families consisting of designated glycan features.

### Examples

### Material and Methods:

### Example 1: Identification of proteins containing carbohydrate recognition domains (CRDs).

Mouse lectin sequences were obtained using a domain-based approach. Briefly, proteins with a C-type lectin-like/IPR001304 domain were downloaded from InterPro 66.0 and supplemented with proteins obtained in jackhmmer searches using the PF00059.20 lectin C-type domain definition versus the mouse-specific UniProt and Ensembl databases. The collected set of candidate mouse lectins was made non-redundant using nrdb 3.0. The C-type lectin-like regions were extracted from the full-length proteins using the SMART CLECT domain definition with hmmersearch v3.1b2 and extended by 5 amino acids on both sides. To reduce redundancy, principal isoforms were selected using appris 2016_10.v24. In addition, the CRD domains for Galectins and Siglecs were added. In case a gene contained more than 1 CRD, all CRDs were cloned separately and differentiated by _1, _2, etc.

### Example 2: Cloning of C-type lectin expression vectors.

We used the pCAGG_00_ccb plasmid and removed the toxic ccb element by cleaving the plasmid with BsaI. Thereafter, we inserted a Fc-fusion construct, consisting of the IL2 secretion signal, followed by an EcoRV restriction site, a (GGGS)₃ linker domain and the mouse IgG2a-Fc domain. Subsequently, each identified CRD, was cloned in-frame into the EcoRV site.

### Example 3: Transfection and purification of the lectin-mIgG2a fusion proteins.

The CRD containing plasmids were transfected into Free-style^{™} 293-F cells. Briefly, the day before transfection, 293-F cells were diluted to 0.7x10⁶ cells/ml in 30 ml Freestyle^{™} 293-F medium and grown at 120 rpm at 37°C with 8% CO2. The next morning, 2 µl polyethylenimine (PEI) 25K (1mg/ml; Polysciences, 23966-1) per µg of plasmid DNA were mixed with pre-warmed Opti-MEM media (ThermoFisher Scientific, 31985-062) to a final volume of 950 µl in tube A. In tube B, 1 µg of DNA per ml of media was mixed with pre-warmed Opti-MEM to a final volume of 950 µl. Then, the contents of tube A and B were mixed, vortexed for 1 min and incubated at room temperature for 15 min. Thereafter, the transfection mixture was added to the cell suspension. 24h after transfection, EX-CELL 293 Serum-Free Medium (Sigma Aldrich, 14571C) was added to a final concentration of 20%. The transfected cells were grown for 120h and the supernatants, containing the secreted lectin-mIgG2a fusion proteins, harvested by centrifugation at 250g for 10 min.

Purification of the mouse lectin-mIgG2a fusion proteins was performed using Protein A agarose resin (Gold Biotechnology, P-400-5). The protein A beads were pelleted at 150g for 5 min and washed once with 1x binding buffer (0.02 M Sodium Phosphate, 0.02% sodium azide, pH=7.0), before resuspending in 1x binding buffer. Immediately preceding purification, aggregates were pelleted from the cell culture supernatant by centrifugation for 10 min at 3000g. 10x binding buffer was added to the cell culture supernatant to a final concentration of 1x as well as 4 µl of protein A beads per ml of cell culture supernatant. The bead/ supernatant mixture was incubated overnight at 4 °C. The next morning, beads were collected by centrifugation for 5 min at 150g, washed twice with 20 and 10 bead volumes of 1x binding buffer, the bead pellets transferred to a 1ml spin column (G-Biosciences, 786-811) and washed once more with 1 bead volume of 1x binding buffer. Excess buffer was removed by centrifugation at 100g for 5 sec. Lectin-mIgG2a fusion proteins were eluted from the protein A beads by resuspending the beads in 1 bead volume of Elution buffer (100mM Glycine-HCl, pH=2-3, 0.02% sodium azide). After 30 sec of incubation the elution buffer was collected into a 2 ml Eppendorf tube, containing Neutralization buffer (1M Tris, pH=9.0, 0.02% sodium azide) by centrifugation at 100g for 15 sec. Elution was performed for a total of 3 times. The 3 eluted fractions were pooled and the protein concentrations measured with the Pierce^{™} BCA Protein Assay Kit (ThermoFisher Scientific, 23225) using the Pierce^{™} Bovine Gamma Globulin Standard (ThermoFisher Scientific, 23212). To confirm the purity of the eluted lectin-IgG fusion proteins, we performed an SDS-PAGE, followed by a Coomassie staining. Briefly, 1 µg of eluted lectin-IgG fusion protein was mixed with Sample Buffer, Laemmli 2x Concentrate (Sigma-Aldrich, S3401) and heated to 95°C for 5 minutes. Thereafter, the samples were loaded onto NuPage^{™} 4-12% Bis-Tris gels (ThermoFisher Scientific, NP0321BOX) and run in 1x MOPS buffer at 140V for 45 minutes. The gel was subsequently stained with InstantBlue^{™} Safe Coomassie Stain (Sigma-Aldrich, ISB1L) for 1h and de-stained with distilled water. The gel picture was acquired with the ChemiDoc MP Imaging System (BioRad) in the Coomassie setting.

### Example 4: Recombinant expression of SARS-CoV-2 Spike protein and the receptor binding domain (RBD).

Recombinant protein expression was performed by transient transfection of HEK293-6E cells, licensed from National Research Council (NRC) of Canada, as previously described (Durocher et al. (2002) Nucleic Acids Res 30, E9). Briefly, HEK293-6E cells were cultivated in FreeStyle F17 expression medium (Thermo Fisher Scientific, A1383502) supplemented with 0.1% (v/v) Pluronic F-68 (Thermo Fisher Scientific, 24040032) and 4 mM L-glutamine (Thermo Fisher Scientific, 25030081) in shaking flasks at 37°C, 8% CO₂, 80% humidity and 130 rpm in a Climo-Shaker ISF1-XC (Adolf Kühner AG). The pCAGGS vector constructs, containing either the sequence of the SARS-CoV-2 RBD (residues R319-F541) or the complete luminal domain of the Spike protein (modified by removing all arginine (R) residues from the polybasic furin cleavage site RRAR and introduction of the stabilizing point mutations K986P and V987P) were kindly provided by Florian Krammer, Icahn School of Medicine at Mount Sinai (NY, United States) (Amanat et al. (2020) Nature Medicine 26, 1033-1036; Stadlbauer et al. (2020) Curr Protoc Microbiol 57, e100). Transient transfection of the cells was performed at a cell density of approximately 1.7×10⁶ cells/mL culture volume using a total of 1 µg of plasmid DNA and 2 µg of linear 40-kDa PEI (Polysciences, 24765-1) per mL culture volume. 48 h and 96 h after transfection, cells were supplemented with 0.5% (w/v) tryptone N1 (Organotechnie 19553) and 0.25% (w/v) D(+)-glucose (Carl Roth X997.1). Soluble proteins were harvested after 120-144 h by centrifugation (10 000 g, 15 min, 4°C).

### Example 5: Purification of recombinant trimeric Spike protein and monomeric RBD of SARS-CoV-2.

For purification, the supernatants were filtered through 0.45 µm membrane filters (Merck Millipore HAWP04700), concentrated and diafiltrated against 20 mM phosphate buffer containing 500 mM NaCl and 20 mM imidazole (pH 7.4) using a Labscale TFF system equipped with a 5 kDa cut-off Pellicon^{™} XL device (Merck Millipore, PXC005C50). His-tagged trimer Spike and monomeric RBD were captured using a 5 mL HisTrap FF crude column (Cytiva, 17528601) connected to an ÄKTA pure chromatography system (Cytiva). Bound proteins were eluted by applying a linear gradient of 20 to 500 mM imidazole over 20 column volumes. Fractions containing the protein of interest were pooled, concentrated using Vivaspin 20 Ultrafiltration Units (Sartorius, VS2011) and dialyzed against PBS (pH 7.4) at 4°C overnight using a SnakeSkin Dialysis Tubing (Thermo Fisher Scientific, 68100). The RBD was further polished by size exclusion chromatography (SEC) using a HiLoad 16/600 Superdex 200 pg column (Cytiva, 28-9893-35) equilibrated with PBS (pH 7.4). Both purified proteins were stored at -80°C until further use.

### Example 6: Glycoproteomic analysis of Spike and RBD.

Peptide mapping and glycoproteomic analysis of all samples were performed on in-solution proteolytic digests of the respective proteins by LC-ESI-MS(/MS). In brief, the pH of the samples was adjusted to pH 7.8 by the addition of 1 M HEPES, pH 7.8 to a final concentration of 100 mM HEPES, pH 7.8. The samples were then chemically reduced and S-alkylated using a final concentration of 10 mM dithiothreitol for 30 min at 56°C and a final concentration of 20 mM iodoacetamide for 30 min at room-temperature in the dark, respectively. To maximize protein sequence-coverage of the analysis, proteins were digested with either Trypsin (Promega), a combination of Trypsin and GluC (Promega) or Chymotrypsin (Roche). Eventually, all proteolytic digests were acidified by addition of 10% formic acid to pH 2 and directly analyzed by LC-ESI-MS(/MS) using an a capillary BioBasic C18 reversed-phase column (BioBasic-18, 150 x 0.32 mm, 5 µm, Thermo Scientific), installed in an Ultimate U3000 HPLC system (Dionex), developing a linear gradient from 95% eluent A (80 mM ammonium formate, pH 3.0, in HPLC-grade water) to 65% eluent B (80% acetonitrile in 80 mM ammonium formate, pH 3.0) over 50 min, followed by a linear gradient from 65% to 99% eluent B over 15 min, at a constant flow rate of 6 µL/min, coupled to a maXis 4G Q-TOF instrument (Bruker Daltonics; equipped with the standard ESI source). For (glyco)peptide detection and identification, the mass-spectrometer was operated in positive ion DDA mode (i.e. switching to MS/MS mode for eluting peaks), recording MS-scans in the m/z range from 150 to 2200 Th, with the 6 highest signals selected for MS/MS fragmentation. Instrument calibration was performed using a commercial ESI calibration mixture (Agilent). Site-specific profiling of protein glycosylation was performed using the dedicated Q-TOF data-analysis software packages Data Analyst (Bruker Daltonics) and Protein Scape (Bruker Daltonics), in conjunction with the MS/MS search engine MASCOT (Matrix Sciences Ltd.) for automated peptide identification.

### Example 7: ELISA assays to detect lectin binding to Spike and RBD.

Briefly, 50 µl of full-length Spike-H6 (4 µg/ml, purified from HEK, diluted in PBS), RBD-H6 (2 µg/ml, purified from HEK, diluted in PBS) or human recombinant soluble ACE2 (hrsACE2; 4 µg/ml, purified from CHO, diluted in PBS, see Monteil et al. (Cell 181, 2020: 905-913 e907), per well were used to coat a clear flat-bottom MaxiSorp 96-well plate (Thermo Fisher Scientific, 442404) for 2h at 37°C. Thereafter, the coating solution was discarded and the plate was washed 3 times with 300 µl of wash buffer (1xTBS, 1mM CaCl₂, 2mM MgCl₂, 0.25% Triton X-100 (Sigma-Aldrich, T8787)). Unspecific binding was blocked with 300 µl of blocking buffer (1xTBS, 1% BSA Fraction V (Applichem, A1391,0100), 1mM CaCl₂, 2mM MgCl₂ and 0.1% Tween-20 (Sigma-Aldrich, P1379)) for 30 min at 37°C. After removal of the blocking solution, 50 µl of either the mouse lectin-mIgG2a (10 µg/ml, diluted in blocking buffer), human CD209-hIgG1 (R&D Systems, 161-DC-050), human CD299-hIgG1 (R&D Systems, 162-D2-050), human CLEC4G-hIgG1 (Acro Biosystems, CLG-H5250-50ug) (10 µg/ml, diluted in blocking buffer), recombinant human ACE2-mIgG1 (2 µg/ml, diluted in blocking buffer, Sino Biological, 10108-H05H) or recombinant human ACE2-hIgG1 (2 µg/ml, diluted in blocking buffer, Sino Biological, 10108-H02H) were added for 1h at room temperature. After washing for 3 times, 100 µl of 0.2 µg/ml HRP-conjugated goat anti-Mouse IgG (H+L) (Thermo Fisher Scientific, 31430) or goat anti-Human IgG (H+L) (Promega, W4031) antibodies were added for 30 min at room temperature. Subsequently, plates were washed as described above. To detect binding, 1 tablet of OPD substrate (Thermo Fisher Scientific, 34006) was dissolved in 9 ml of deionized water and 1ml of 10x Pierce^{™} Stable Peroxide Substrate Buffer (Thermo Fisher, Scientific, 34062). 100 µl of OPD substrate solution were added per well and incubated for 15 min at room temperature. The reaction was stopped by adding 75 µl of 2.5M sulfuric acid and absorption was read at 490 nm. Absorption was measured for each lectin-Fc fusion protein tested against full-length Spike-H6, RBD-H6 or hrsACE2 and normalized against bovine serum albumin coated control wells.

### Example 8: Protein denaturation and removal of N-glycans.

To denature the full-length Spike-H6, 10 mM DTT was added to 40 µg/ml of protein. The samples were incubated at 85°C for 10 min. Thereafter, the denatured proteins were diluted to 4 µg/ml with PBS and a clear flat-bottom Maxisorp 96-well plate was coated with 50 µl per well for 2h at 37°C. To remove the *N*-glycans from the full-length Spike-H6, 0.2 µg protein were denatured as above and adjusted to a final concentration of 1x Glycobuffer 2 containing 125U PNGase F per µg (NEB, P0704S) in 50 µl. After incubation for 2h at 37°C, the reaction was stopped by heat-inactivation for 10 min at 75°C. Spike proteins were then diluted to 4 µg/ml with PBS and a clear flat-bottom Maxisorp 96-well plate was coated with 50 µl per well for 2h at 37°C. ELISA protocols were performed as described above. To confirm the deglycosylation of Spike proteins, 0.5 µg were loaded on an SDS-PAGE gel followed by a Coomassie staining.

### Example 9: Surface plasmon resonance (SPR) measurements.

A commercial SPR (BIAcore X, GE Healthcare, USA) was used to study the kinetics of binding and dissociation of lectin-Fc dimers to the trimeric full-length Spike in real time. Spike-H6 was immobilized on a Sensor Chip NTA (Cytiva, BR100034) via its His6-tag after washing the chip for at least 3 minutes with 350 mM EDTA and activation with a 1 min injection of 0.5 mM NiCl₂. 50 nM Spike were injected multiple times to generate a stable surface. For the determination of kinetic and equilibrium constants, the lectin samples (murine Clec4g, murine CD209c, human CLEC4G, human CD209) were injected at different concentrations (10 to 500 nM). As the binding of the lectins is Ca²⁺ dependent, lectins were removed from the surface by washing with degassed calcium free buffer (TBS, 0.1% Tween-20, pH = 7.4). The resonance angle was recorded at a 1 Hz sampling rate in both flow cells and expressed in resonance units (1 RU = 0.0001°). The resulting experimental binding curves were fitted to the "bivalent analyte model", assuming a two-step binding of the lectins to immobilized Spike. All evaluations were done using the BIAevaluation 3.2 software (BIAcore, GE Healthcare, USA).

### Example 10: Single molecule force spectroscopy (SMFS) measurements.

For single molecule force spectroscopy a maleimide-Poly(ethylene glycol) (PEG) linker was attached to 3-aminopropyltriethoxysilane (APTES)-coated atomic force microscopy (AFM) cantilevers by incubating the cantilevers for 2h in 500 µL of chloroform containing 1 mg of maleimide-PEG-N-hydroxysuccinimide (NHS) (Polypure, 21138-2790) and 30 µl of triethylamine. After 3 times washing with chloroform and drying with nitrogen gas, the cantilevers were immersed for 2h in a mixture of 100 µL of 2 mM thiol-trisNTA, 2 µL of 100 mM EDTA (pH 7.5), 5 µL of 1 M HEPES (pH 7.5), 2 µl of 100 mM tris(carboxyethyl)phosphine (TCEP) hydrochloride, and 2.5 µL of 1 M HEPES (pH 9.6) buffer, and subsequently washed with HEPES-buffered saline (HBS). Thereafter, the cantilevers were incubated for 4h in a mixture of 4 µL of 5 mM NiCl₂ and 100 µL of 0.2 µM His-tagged Spike trimers. After washing with HBS, the cantilevers were stored in HBS at 4°C (Oh et al., 2016). For the coupling of lectins to surfaces, a maleimide-PEG linker was attached to an APTES-coated silicon nitride surface. First, 2 µl of 100 mM TCEP, 2 µl of 1M HEPES (pH 9.6), 5 µl of 1M HEPES (pH 7.5), and 2 µl of 100 mM EDTA were added to 100 µl of 200 µg/ml Protein A-Cys (pro-1992-b, Prospec, NJ, USA) in PBS. The surfaces were incubated in this solution for 2h and subsequently washed with PBS and 0.02 M sodium phosphate containing 0.02% sodium azide, pH=7.0. Finally, 100 µl of 200 µg/ml lectin-Fc fusion proteins were added to the surfaces overnight.

Force distance measurements were performed at room temperature (∼25 °C) with 0.01 N/m nominal spring constants (MSCT, Bruker) in TBS buffer containing 1 mM CaCl₂ and 0.1 % TWEEN-20. Spring constants of AFM cantilevers were determined by measuring the thermally-driven mean-square bending of the cantilever using the equipartition theorem in an ambient environment. The deflection sensitivity was calculated from the slope of the force-distance curves recorded on a bare silicon substrate. Determined spring constants ranged from 0.008 to 0.015 N/m. Force-distance curves were acquired by recording at least 1000 curves with vertical sweep rates between 0.5 and 10 Hz at a z-range of typically 500 - 1000 nm (resulting in loading rates from 10 to 10,000 pN/s), using a commercial AFM (5500, Agilent Technologies, USA). The relationship between experimentally measured unbinding forces and parameters from the interaction potential were described by the kinetic models of Bell (Bell, 1978) and Evans and Ritchie (Evans and Ritchie, 1997). In addition, multiple parallel bond formation was calculated by the Williams model (Williams, 2003) from the parameters derived from single bond analysis. The binding probability was calculated from the number of force experiments displaying unbinding events over the total number of force experiments.

The probability density function (PDF) of unbinding force was constructed from unbinding events at the same pulling speed. For each unbinding force value, a Gaussian unitary area was computed with its center representing the unbinding force and the width (standard deviation) reflecting its measuring uncertainty (square root of the variance of the noise in the force curve). All Gaussian areas from one experimental setting were accordingly summed up and normalized with its binding activity to yield the experimental PDF of unbinding force. PDFs are equivalents of continuous histograms as shown in Fig. 3C.

### Example 11: High-speed AFM (hsAFM) and data analysis.

Purified SARS-CoV-2 trimeric Spike glycoproteins, murine Clec4g and CD209c and hCLEC4g and hCD209 were diluted to 20 µg/ml with imaging buffer (20mM HEPES, 1mM CaCl₂, pH 7.4) and 1.5 µl of the protein solution was applied onto freshly cleaved mica discs with diameters of 1.5 mm. After 3 minutes, the surface was rinsed with ∼15µL imaging buffer (without drying) and the sample was mounted into the imaging chamber of the hsAFM (custom-built, RIBM, Japan). Pictures for movies were captured in imaging buffer containing 3µg/ml of either Clec4g, CD209c, hCLEC4G or hCD209. An ultra-short cantilever (USC-F1.2-k0.15 nominal spring constant 0.15 N/m, Nanoworld, Switzerland) was used and areas of 100x100nm containing single molecules were selected to capture the hsAFM movies at a scan rate of ∼150-300 ms per frame. During the acquisition of the movies, the amplitude was kept constant and set to 90-85% of the free amplitude (typically ∼3 nm). Data analysis was performed using the Gwyddion 2.55 software. Images were processed to remove background and transient noise. For volume measurements, a height threshold mask was applied over the protein structures with a minimum height of 0.25 - 0.35 nm to avoid excessive background noise in the masked area. The numbers of lectin molecules bound to the Spike trimers was calculated based on the measured mean volumes of the full-length Spike, the lectins, and the Spike-lectin complexes, averaged over the recorded time-periods.

### Example 12: AFM measured Spike binding to Vero E6 cells.

Vero E6 cells were grown on culture dishes using DMEM containing 10% FBS, 500 units/mL penicillin and 100 µg/mL streptomycin, at 37°C with 5% CO₂. For AFM measurements, the cell density was adjusted to about 10-30% confluency. Before the measurements, the growth medium was exchanged to a physiological HEPES buffer containing 140 mM NaCl, 5 mM KCl, 1 mM MgCl₂, 1 mM CaCl₂, and 10 mM HEPES (pH 7.4). Lectins were added at the indicated concentrations. Using a full-length Spike trimer anchored to an AFM cantilever (described above), force-distance curves were recorded at room temperature on living cells with the assistance of a CCD camera for localization of the cantilever tip on selected cells. The sweep range was fixed at 3000 nm and the sweep rate was set at 1 Hz. For each cell, at least 100 force-distance cycles with 2000 data points per cycle and a typical force limit of about 30 pN were recorded.

### Example 13: Structural modelling.

Structural models of the SARS-CoV-2 Spike protein were based on the model of the fully glycosylated Spike-hACE2 complex.

Experimental structures deposited in the protein databank (PDB) were used to model the complex that is formed by the binding of SARS-CoV-2 Spike and ACE2 (Walls et al. (2020) Cell 181, 281-292 e286; Yan et al. (2020) Science 367, 1444-1448). RBD domain in complex with ACE2 was superimposed with Spike with one open RBD domain (PDB: 6VYB) and SWISS-MODEL (Waterhouse et al. (2018) Nucleic Acids Res 46, W296-W303) was used to model missing residues in Spike (GenBank QHD43416.1). Glycan structures were added using the methodology outlined by Turupcu et al. (FEMS Microbiol Rev 38, 2014: 598-632). For hCLEC4G a homology model of residues 118 - 293 was constructed using Swiss-Model using residues 4 - 180 of chain A of the crystal structure of the carbohydrate recognition domain of DC-SIGNR (CD299) (PDB entry 1sl6). This fragment shows a sequence identity of 36% with hCLEC4G, and the resulting model showed an overall QMEAN value of -2.68. For mClec4g, the model consisted of residues 118 - 294, with a sequence identity of 38 % to the same template model. The resulting QMEAN value was -2.88. A calcium ion and the bound Lewis x oligosaccharide of the template were taken over into the model, indicating the location of the carbohydrate binding site. For hCD209, the crystal structure of the carbohydrate recognition domain of CD209 (DC-SIGN) complexed with Man4 (PDB entry 1sl4) was used. To identify binding sites of hCLEC4G and hCD209 to the Spike-hACE2 complex, a superposition of the bound carbohydrates with the glycans on Spike was performed. For hCLEC4G, we used the complex glycans at N343 of the third monomer of Spike, with the receptor binding domain in an 'up' position, while N343 glycans on monomer 1 and 2 were modelled with the receptor binding domain in a 'down' position. For hCD209 we used the high-mannose glycan at position N234 in monomer 1-3 of Spike, respectively. These glycan structures were chosen in accordance with the full-length Spike glycoproteome.

### Example 14: SARS-CoV-2 infections.

Vero E6 cells were seeded in 48-well plates (5x10⁴ cells per well) in DMEM containing 10% FBS. 24 hours post-seeding, different concentrations of lectins were mixed with 10³ PFU of virus (1:1) to a final volume of 100µl per well in DMEM (resulting in a final concentration of 5% FBS). After incubation for 30 min at 37°C, Vero E6 were infected either with mixes containing lectins/SARS-CoV-2, SARS-CoV-2 alone, or mock infected. 15 hours post-infection, supernatants were removed, cells were washed 3 times with PBS and then lysed using Trizol Reagent (Thermo Fisher Scientific, 15596026). The qRT-PCR for the detection of viral RNA was performed as previously described (Monteil et al., 2020, supra). Briefly, RNA was extracted using the Direct-zol RNA MiniPrep kit (Zymo Research, R2051). The qRT-PCR was performed for the SARS-CoV-2 E gene and RNase P was used as an endogenous gene control to normalize viral RNA levels to the cell number. Lectins were independently tested for cellular toxicity in an ATP-dependent assay (Cell-Titer Glo, Promega) and cells found to exhibit >80% viability up to a concentration of 200 µg/ml.

The following PCR Primers were used:
SARS-CoV2 E-gene:
   Forward primer: 5'-ACAGGTACGTTAATAGTTAATAGCGT-3' (SEQ ID NO: 3)
   Reverse primer: 5'-ATATTGCAGCAGTACGCACACA-3' (SEQ ID NO: 4)
   Probe: FAM-ACACTAGCCATCCTTACTGCGCTTCG-QSY (SEQ ID NO: 5)
RNase P:
   Forward primer: 5'-AGATTTGGACCTGCGAGCG-3' (SEQ ID NO: 6)
   Reverse primer 5'-GAGCGGCTGTCTCCACAAGT-3' (SEQ ID NO: 7)
   Probe: FAM-TTCTGACCTGAAGGCTCTGCGCG-MGB (SEQ ID NO: 8)

### Results:

### Example 15: Preparation of the first near genome-wide lectin library to screen for novel binders of Spike glycosylation.

To systematically identify lectins that bind to the trimeric Spike protein and RBD of SARS-CoV-2, we searched for all annotated carbohydrate recognition domains (CRDs) of mouse C-type lectins, Galectins and Siglecs. Of 168 annotated CRDs, we were able to clone, express and purify 143 lectin-CRDs as IgG2a-Fc fusion proteins from human HEK293F cells (Fig. 1A, table 1). The resulting dimeric lectin-Fc fusion proteins (hereafter referred to as lectins) showed a high degree of purity (Fig. 1B). This collection of lectins is, to our knowledge, the first comprehensive library of mammalian CRDs.

**Table 1. Overview of carbohydrate recognition domains (CRDs) used for the lectin library**

| This table presents a list of CRDs expressed and purified as Fc-fusion proteins for the lectin library. Information displayed are the lectin name, the family and in the case of C-type lectins, the group and group name the CRD belongs to. CRDs from lectin that contain several CRDs are distinguished by suffix numbers. CTL = C-type lectin | | | |
|---|---|---|---|
| **Family** | **CTL Group** | **CTL Group Name** | **Lectin** |
| C-type Lectin | Group I | Proteoglycans | Acan |
| C-type Lectin | Group I | Proteoglycans | Bcan |
| C-type Lectin | Group I | Proteoglycans | Ncan |
| C-type Lectin | Group I | Proteoglycans | Vcan |
| C-type Lectin | Group II | Type II receptors | Asgr1 |
| C-type Lectin | Group II | Type II receptors | Asgr2 |
| C-type Lectin | Group II | Type II receptors | Cd207 |
| C-type Lectin | Group II | Type II receptors | Cd209a |
| C-type Lectin | Group II | Type II receptors | Cd209b |
| C-type Lectin | Group II | Type II receptors | Cd209c |
| C-type Lectin | Group II | Type II receptors | Cd209d |
| C-type Lectin | Group II | Type II receptors | Cd209e |
| C-type Lectin | Group II | Type II receptors | Cd209f |
| C-type Lectin | Group II | Type II receptors | Cd209g |
| C-type Lectin | Group II | Type II receptors | CleclOa |
| C-type Lectin | Group II | Type II receptors | Clec4a |
| C-type Lectin | Group II | Type II receptors | Clec4a1 |
| C-type Lectin | Group II | Type II receptors | Clec4a3 |
| C-type Lectin | Group II | Type II receptors | Clec4a4 |
| C-type Lectin | Group II | Type II receptors | Clec4b1 |
| C-type Lectin | Group II | Type II receptors | Clec4e |
| C-type Lectin | Group II | Type II receptors | Clec4f |
| C-type Lectin | Group II | Type II receptors | Clec6a |
| C-type Lectin | Group II | Type II receptors | Colec12 |
| C-type Lectin | Group II | Type II receptors | Mgl2 |
| C-type Lectin | Group II | Type II receptors | Clec4b2 |
| C-type Lectin | Group II | Type II receptors | Clec4g |
| C-type Lectin | Group II | Type II receptors | Fcer2a |
| C-type Lectin | Group III | Collectins | Mbl1 |
| C-type Lectin | Group III | Collectins | Mbl2 |
| C-type Lectin | Group III | Collectins | Sftpal |
| C-type Lectin | Group III | Collectins | Sftpd |
| C-type Lectin | Group III | Collectins | Colec10 |
| C-type Lectin | Group III | Collectins | Colec11 |
| C-type Lectin | Group IV | Select ins | Sele |
| C-type Lectin | Group IV | Select ins | Sell |
| C-type Lectin | Group IV | Select ins | Selp |
| C-type Lectin | Group V | NK cell receptors | Cd69 |
| C-type Lectin | Group V | NK cell receptors | Cd72 |
| C-type Lectin | Group V | NK cell receptors | Clec1a |
| C-type Lectin | Group V | NK cell receptors | Clec1b |
| C-type Lectin | Group V | NK cell receptors | Clec2d |
| C-type Lectin | Group V | NK cell receptors | Clec2e |
| C-type Lectin | Group V | NK cell receptors | Clec2f |
| C-type Lectin | Group V | NK cell receptors | Clec2g |
| C-type Lectin | Group V | NK cell receptors | Clec2h |
| C-type Lectin | Group V | NK cell receptors | Clec2i |
| C-type Lectin | Group V | NK cell receptors | Clec2l |
| C-type Lectin | Group V | NK cell receptors | Clec5a |
| C-type Lectin | Group V | NK cell receptors | Clec7a |
| C-type Lectin | Group V | NK cell receptors | Clec9a |
| C-type Lectin | Group V | NK cell receptors | Klrc1 |
| C-type Lectin | Group V | NK cell receptors | Klrc2 |
| C-type Lectin | Group V | NK cell receptors | Klrc3 |
| C-type Lectin | Group V | NK cell receptors | Klrd1 |
| C-type Lectin | Group V | NK cell receptors | Klre1 |
| C-type Lectin | Group V | NK cell receptors | Klrg1 |
| C-type Lectin | Group V | NK cell receptors | Klrg2 |
| C-type Lectin | Group V | NK cell receptors | Klri1 |
| C-type Lectin | Group V | NK cell receptors | Klri2 |
| C-type Lectin | Group V | NK cell receptors | Klrk1 |
| C-type Lectin | Group V | NK cell receptors | Gm156 |
| C-type Lectin | Group V | NK cell receptors | Klrb1 |
| C-type Lectin | Group V | NK cell receptors | Klrb1a |
| C-type Lectin | Group V | NK cell receptors | Klrblb_1 |
| C-type Lectin | Group V | NK cell receptors | Klrblc |
| C-type Lectin | Group V | NK cell receptors | Clec12b |
| C-type Lectin | Group V | NK cell receptors | Clec2j |
| C-type Lectin | Group V | NK cell receptors | Olr1 |
| C-type Lectin | Group VI | Mannose receptor family | Ly75_1 |
| C-type Lectin | Group VI | Mannose receptor family | Ly75 _10 |
| C-type Lectin | Group VI | Mannose receptor family | Ly75_2 |
| C-type Lectin | Group VI | Mannose receptor family | Ly75_3 |
| C-type Lectin | Group VI | Mannose receptor family | Ly75_4 |
| C-type Lectin | Group VI | Mannose receptor family | Ly75_5 |
| C-type Lectin | Group VI | Mannose receptor family | Ly75_6 |
| C-type Lectin | Group VI | Mannose receptor family | Ly75_7 |
| C-type Lectin | Group VI | Mannose receptor family | Ly75_8 |
| C-type Lectin | Group VI | Mannose receptor family | Ly75_9 |
| C-type Lectin | Group VI | Mannose receptor family | Mrc1_1 |
| C-type Lectin | Group VI | Mannose receptor family | Mrc1_2 |
| C-type Lectin | Group VI | Mannose receptor family | Mrc1_3 |
| C-type Lectin | Group VI | Mannose receptor family | Mrc1_5 |
| C-type Lectin | Group VI | Mannose receptor family | Mrc1_6 |
| C-type Lectin | Group VI | Mannose receptor family | Mrc1_7 |
| C-type Lectin | Group VI | Mannose receptor family | Mrc1_8 |
| C-type Lectin | Group VI | Mannose receptor family | Mrc2_2 |
| C-type Lectin | Group VI | Mannose receptor family | Mrc2_3 |
| C-type Lectin | Group VI | Mannose receptor family | Mrc2_5 |
| C-type Lectin | Group VI | Mannose receptor family | Mrc2_6 |
| C-type Lectin | Group VI | Mannose receptor family | Mrc2_7 |
| C-type Lectin | Group VI | Mannose receptor family | Mrc2_8 |
| C-type Lectin | Group VI | Mannose receptor family | Pla2r1_1 |
| C-type Lectin | Group VI | Mannose receptor family | Pla2r1_2 |
| C-type Lectin | Group VI | Mannose receptor family | Pla2r1_4 |
| C-type Lectin | Group VI | Mannose receptor family | Pla2r1_ 5 |
| C-type Lectin | Group VI | Mannose receptor family | Pla2r1_7 |
| C-type Lectin | Group VI | Mannose receptor family | Pla2r1_ 8 |
| C-type Lectin | Group VII | Reg group | Reg1 |
| C-type Lectin | Group VII | Reg group | Reg2 |
| C-type Lectin | Group VII | Reg group | Reg3a |
| C-type Lectin | Group VII | Reg group | Reg3b |
| C-type Lectin | Group VII | Reg group | Reg3d |
| C-type Lectin | Group VII | Reg group | Reg3g |
| C-type Lectin | Group VIII | Simple type I receptors | Layn |
| C-type Lectin | Group VIII | Simple type I receptors | Chodl |
| C-type Lectin | Group IX | Tetranectin | Clec11a |
| C-type Lectin | Group IX | Tetranectin | Clec3b |
| C-type Lectin | Group IX | Tetranectin | Clec3a |
| C-type Lectin | Group X | Polycystin | Pkd1 |
| C-type Lectin | Group X | Polycystin | Pkd1l2 |
| C-type Lectin | Group X | Polycystin | Pkd1l3 |
| C-type Lectin | Group XI | Attractin | Atrn |
| C-type Lectin | Group XI | Attractin | Atrnll |
| C-type Lectin | Group XII | EMBP group | Prg2 |
| C-type Lectin | Group XII | EMBP group | Prg3 |
| C-type Lectin | Group XIII | IDD | Dgcr2 |
| C-type Lectin | Group XIV | Endosialin family | Cd93 |
| C-type Lectin | Group XIV | Endosialin family | Clec14a |
| C-type Lectin | Group XIV | Endosialin family | Thbd |
| C-type Lectin | Group XV | Bimlec | Cd302 |
| C-type Lectin | Group XVI | CL18A | Clec18a |
| C-type Lectin | Group XVII | Proteoglycan | Frem1 |
| Galectin | | | Grifin |
| Galectin | | | Lgals12_2 |
| Galectin | | | Lgals2 |
| Galectin | | | Lgals4 1 |
| Galectin | | | Lgals4 2 |
| Galectin | | | Lgals6 2 |
| Galectin | | | Lgals8 1 |
| Galectin | | | Lgals8 2 |
| Galectin | | | Lgals9 2 |
| Galectin | | | Lgalsl |
| Galectin | | | Lgalslb |
| Siglec | | | Cd22 |
| Siglec | | | Cd33 |
| Siglec | | | Mag |
| Siglec | | | Siglecl |
| Siglec | | | Siglec10 |
| Siglec | | | Siglec12 |
| Siglec | | | Siglec15 |
| Siglec | | | Siglec5 |
| Siglec | | | Siglech |

We next recombinantly expressed monomeric RBD and full-length trimeric Spike protein (hereafter referred to as Spike protein) in human HEK293-6E cells. Using mass spectrometry, we characterized all 22 *N*-glycosylation sites on the full-length Spike protein and 2 *N*-glycosylation sites on the RBD (Fig. 1C and 10). Most of the identified structures were in accordance with previous studies using full-length Spike (Watanabe et al. (2020) Science 369, 330-333) with the exception of N331, N603 and N1194, which presented a higher structural variability of the glycan branches (Fig. 1C and 10). The detected *N*-glycan species ranged from poorly processed oligo-mannose structures to highly processed multi-antennary complex *N*-glycans in a site-dependent manner. This entailed also a large variety of terminal glycan epitopes, which could act as ligands for lectins. Notably, the two glycosylation sites N331 and N343 located in the RBD carried more extended glycans, including sialylated and di-fucosylated structures, when expressed as an independent construct as opposed to the full-length Spike protein (Fig. 1C and 10). These data underline the complex glycosylation of Spike and reveal that *N*-glycosylation of the RBD within the 3D context of full-length trimeric Spike is different from *N*-glycosylation of the RBD expressed as minimal ACE2 binding domain.

### Example 16: CD209c and Clec4g are novel high affinity binders of SARS-CoV-2 Spike.

We evaluated the reactivity of our murine lectin library against the trimeric Spike and monomeric RBD of SARS-CoV-2 using an ELISA assay (fig. 6A). This screen revealed that CD209c (SIGNR2), Clec4g (LSECtin), and Reg1 exhibited pronounced binding to Spike, whereas Mgl2 and Asgr1 displayed elevated binding to the RBD (Fig. 2A, B and table 2). Further, we investigated the reactivity of the lectin library against human recombinant soluble ACE2 (hrsACE2); none of the lectins bound to hrsACE2 (fig. 6B). We excluded Reg1 from further studies due to inconsistent ELISA results, likely due to protein instability. Asgr1 was excluded because it bound only to RBD but not to the Spike trimer, in accordance to the differences in glycosylation of glyco-sites N331 and N343 between Spike and RBD (Fig. 1C). This highlights the importance of using a full-length trimeric Spike protein for functional studies. To confirm that the observed interactions were independent of protein conformation, Spike was denatured prior to the ELISA assay; binding of CD209c and Clec4g to the unfolded Spike remained unaltered (Fig. 2C). Importantly, enzymatic removal of *N*-glycans by PNGase F treatment reduced the binding of CD209c, Clec4g, and Mgl2 towards Spike (Fig. 2D and 6C), confirming *N*-glycans as ligands. Binding of ACE2, which relies on protein-protein interactions, was completely abrogated when Spike was denatured (Fig. 2C). These data identify lectins that have the potential to bind to the RBD and trimeric Spike of SARS-CoV-2.

**Table 2. ELISA screen of the lectin-Fc library against SARS-CoV-2 Spike, RBD and hrsACE2**

| Results from the ELISA screens of the lectin-Fc library against indicated targets. Data displayed per lectin is mean and standard deviation (SD) (n=2). SD=NA indicates that mean was calculated from a single replicate only. | | | | | | |
|---|---|---|---|---|---|---|
| | **Full-length Spike (n=2)** | | **RBD Spike (n=2)** | | **hrsACE2 (n=2)** | |
| **Name** | **Mean** | **SD** | **Mean** | **SD** | **Mean** | **SD** |
| Acan | 0,023 | 0,000 | 0,035 | 0,004 | 0,081 | 0,036 |
| Asgr1 | 0,121 | 0,001 | 0,697 | 0,135 | 0,004 | 0,002 |
| Asgr2 | 0,003 | 0,000 | 0,013 | 0,002 | 0,034 | 0,014 |
| Atrn | 0,002 | 0,000 | 0,001 | 0,001 | 0,002 | 0,004 |
| Atrnl1 | 0,001 | 0,000 | 0,001 | 0,000 | -0,003 | 0,007 |
| Bcan | -0,111 | 0,018 | 0,101 | 0,101 | -0,538 | 0,062 |
| Blank | 0,000 | 0,003 | 0,000 | 0,001 | -0,002 | 0,003 |
| Cd207 | 0,028 | 0,010 | 0,118 | 0,052 | 0,123 | 0,032 |
| Cd209a | 0,014 | 0,002 | 0,028 | 0,010 | 0,049 | 0,035 |
| Cd209b | -0,032 | 0,062 | 0,113 | 0,055 | -0,094 | 0,036 |
| Cd209c | 2,049 | 0,087 | 0,430 | 0,074 | -0,403 | 0,056 |
| Cd209d | 0,010 | 0,001 | 0,019 | 0,003 | 0,039 | 0,009 |
| Cd209e | 0,045 | 0,008 | 0,048 | 0,018 | -0,016 | 0,005 |
| Cd209f | 0,018 | 0,000 | 0,015 | 0,002 | 0,182 | 0,001 |
| Cd209g | 0,047 | 0,004 | 0,019 | 0,005 | 0,256 | 0,051 |
| Cd22 | 0,027 | 0,001 | 0,004 | 0,001 | 0,006 | 0,007 |
| Cd302 | -0,002 | 0,000 | 0,001 | 0,001 | -0,002 | 0,002 |
| Cd33 | 0,023 | 0,001 | 0,009 | 0,002 | 0,216 | 0,027 |
| Cd69 | 0,001 | 0,001 | 0,001 | 0,000 | -0,022 | 0,002 |
| Cd72 | 0,033 | 0,002 | 0,015 | 0,002 | 0,044 | 0,016 |
| Cd93 | 0,000 | 0,000 | -0,005 | 0,002 | -0,163 | 0,012 |
| Chodl | 0,002 | 0,000 | 0,001 | 0,000 | -0,001 | 0,005 |
| Clec10a | 0,069 | 0,006 | 0,064 | 0,006 | 0,125 | 0,040 |
| Clec11a | 0,032 | 0,002 | 0,011 | 0,003 | 0,170 | 0,007 |
| Clec12b | 0,002 | 0,001 | 0,001 | NA | 0,007 | 0,012 |
| Clec14a | 0,012 | 0,001 | 0,004 | 0,004 | 0,029 | 0,003 |
| Clec18a | 0,022 | 0,000 | 0,000 | 0,001 | -0,013 | 0,012 |
| Clec1a | -0,005 | 0,001 | 0,010 | 0,009 | -0,006 | 0,006 |
| Clec1b | 0,024 | 0,007 | 0,030 | 0,013 | 0,040 | 0,013 |
| Clec2d | -0,106 | 0,005 | 0,024 | 0,063 | -0,506 | 0,048 |
| Clec2e | 0,017 | 0,000 | 0,016 | 0,003 | 0,066 | 0,016 |
| Clec2f | -0,020 | 0,005 | 0,045 | 0,024 | -0,261 | 0,041 |
| Clec2g | -0,241 | 0,113 | 0,421 | 0,213 | -0,722 | 0,128 |
| Clec2h | -0,387 | 0,002 | 0,237 | 0,284 | -0,707 | 0,141 |
| Clec2i | -0,610 | 0,062 | 0,178 | 0,255 | -0,249 | 0,048 |
| Clec2j | 0,006 | 0,000 | 0,005 | 0,000 | 0,059 | 0,023 |
| Clec2l | -0,001 | 0,000 | 0,000 | 0,000 | 0,000 | 0,001 |
| Clec3a | 0,098 | 0,004 | 0,039 | 0,007 | 0,045 | 0,015 |
| Clec3b | 0,001 | 0,000 | 0,000 | 0,000 | 0,001 | 0,004 |
| Clec4a | 0,014 | 0,003 | 0,036 | 0,020 | 0,062 | 0,023 |
| Clec4a1 | 0,014 | 0,002 | 0,024 | 0,011 | -0,004 | 0,029 |
| Clec4a3 | 0,002 | 0,003 | 0,002 | 0,000 | 0,001 | 0,002 |
| Clec4a4 | 0,004 | 0,001 | 0,007 | 0,000 | -0,009 | 0,003 |
| Clec4b1 | 0,013 | 0,000 | 0,023 | 0,009 | 0,001 | 0,012 |
| Clec4b2 | -0,013 | 0,003 | 0,003 | 0,001 | 0,008 | 0,009 |
| Clec4e | 0,037 | 0,002 | 0,022 | 0,003 | 0,222 | 0,001 |
| Clec4f | -0,146 | 0,052 | 0,362 | 0,142 | 0,194 | 0,073 |
| Clec4g | 1,684 | 0,003 | 0,451 | 0,116 | 0,040 | 0,001 |
| Clec5a | 0,000 | 0,003 | -0,001 | 0,000 | -0,005 | 0,002 |
| Clec6a | 0,003 | 0,000 | 0,029 | 0,014 | -0,032 | 0,016 |
| Clec7a | 0,000 | 0,001 | 0,004 | 0,005 | -0,001 | 0,004 |
| Clec9a | 0,029 | 0,005 | 0,062 | 0,037 | 0,222 | 0,020 |
| Colec10 | 0,036 | 0,004 | 0,008 | 0,001 | 0,185 | 0,014 |
| Colec11 | 0,043 | 0,008 | 0,013 | 0,002 | 0,153 | 0,026 |
| Colec12 | 0,040 | 0,002 | 0,027 | 0,008 | 0,317 | 0,005 |
| Dgcr2 | 0,005 | 0,001 | 0,003 | 0,001 | 0,012 | 0,011 |
| Fcer2a | 0,007 | 0,009 | 0,000 | 0,000 | -0,004 | 0,004 |
| Frem1 | 0,030 | 0,008 | 0,013 | 0,002 | 0,028 | 0,025 |
| Gm156 | 0,074 | 0,005 | 0,048 | 0,005 | 0,009 | 0,015 |
| Grifin | 0,004 | 0,000 | 0,000 | 0,000 | 0,035 | 0,044 |
| IgG2a | 0,001 | 0,002 | 0,000 | 0,001 | -0,003 | 0,005 |
| Klrb1 | 0,002 | 0,001 | 0,005 | 0,004 | 0,012 | 0,009 |
| Klrb1a | 0,000 | 0,000 | 0,002 | 0,000 | 0,000 | 0,005 |
| Klrb1b_1 | 0,001 | 0,000 | 0,002 | NA | 0,003 | 0,005 |
| Klrb1c | 0,001 | 0,000 | 0,004 | 0,004 | -0,004 | 0,001 |
| Klrc1 | 0,006 | 0,002 | 0,010 | 0,001 | 0,039 | 0,003 |
| Klrc2 | 0,025 | 0,007 | 0,035 | 0,006 | 0,104 | 0,006 |
| Klrc3 | -0,007 | 0,003 | 0,061 | 0,064 | 0,086 | 0,045 |
| Klrd1 | 0,003 | 0,001 | 0,004 | 0,001 | 0,006 | 0,012 |
| Klre1 | -0,002 | 0,004 | 0,045 | 0,023 | -0,006 | 0,113 |
| Klrg1 | 0,003 | 0,001 | 0,003 | 0,010 | -0,843 | 0,026 |
| Klrg2 | -0,044 | 0,000 | 0,043 | 0,029 | -0,013 | 0,005 |
| Klri1 | 0,002 | 0,003 | 0,001 | 0,000 | -0,003 | 0,004 |
| Klri2 | 0,006 | 0,005 | 0,001 | 0,000 | 0,005 | 0,010 |
| Klrk1 | 0,000 | 0,001 | 0,000 | 0,000 | -0,003 | 0,002 |
| Layn | 0,002 | 0,000 | 0,000 | 0,000 | -0,005 | 0,010 |
| Lgals12_2 | 0,025 | 0,002 | 0,009 | 0,004 | 0,031 | 0,007 |
| Lgals2 | 0,035 | 0,002 | 0,018 | 0,003 | 0,031 | 0,004 |
| Lgals4_1 | 0,152 | 0,002 | 0,143 | 0,012 | 0,200 | 0,017 |
| Lgals4_2 | 0,034 | 0,000 | 0,014 | 0,003 | 0,031 | 0,008 |
| Lgals6_2 | 0,135 | 0,001 | 0,073 | 0,014 | 0,528 | 0,111 |
| Lgals8_1 | 0,019 | 0,001 | 0,031 | 0,001 | 0,062 | 0,003 |
| Lgals8_2 | 0,037 | 0,002 | 0,012 | 0,003 | 0,074 | 0,025 |
| Lgals9_2 | 0,056 | 0,004 | 0,033 | 0,001 | 0,367 | 0,033 |
| Lgalsl | 0,006 | 0,000 | 0,001 | 0,000 | -0,004 | 0,000 |
| Lgalslb | 0,026 | 0,000 | 0,010 | 0,002 | 0,047 | 0,018 |
| Ly75_1 | -0,434 | 0,401 | 0,290 | 0,390 | -0,571 | 0,242 |
| Ly75 _10 | -0,002 | 0,001 | 0,014 | 0,014 | -0,020 | 0,008 |
| Ly75_2 | -0,319 | 0,035 | -0,206 | 0,013 | -0,135 | 0,003 |
| Ly75_3 | 0,002 | 0,000 | 0,001 | 0,001 | -0,003 | 0,002 |
| Ly75_4 | -0,014 | 0,004 | 0,007 | 0,005 | -0,004 | 0,012 |
| Ly75_5 | -0,170 | 0,078 | 0,140 | 0,087 | -0,106 | 0,045 |
| Ly75_6 | 0,007 | 0,007 | 0,003 | 0,001 | 0,005 | 0,004 |
| Ly75_7 | 0,001 | 0,003 | 0,003 | 0,001 | -0,009 | 0,005 |
| Ly75_8 | 0,011 | 0,002 | 0,007 | 0,005 | 0,054 | 0,005 |
| Ly75_9 | 0,001 | 0,000 | 0,004 | 0,002 | -0,014 | 0,009 |
| Mag | 0,008 | 0,002 | 0,004 | 0,002 | 0,032 | 0,011 |
| Mbl1 | 0,043 | 0,003 | 0,019 | 0,002 | 0,098 | 0,001 |
| Mbl2 | 0,140 | 0,003 | 0,001 | 0,001 | 0,000 | 0,002 |
| Mgl2 | 0,598 | 0,055 | 1,306 | 0,042 | 0,145 | 0,029 |
| Mrc1_1 | 0,109 | 0,059 | 0,076 | 0,015 | 0,161 | 0,042 |
| Mrc1_2 | -0,028 | 0,006 | 0,106 | 0,059 | 0,000 | 0,046 |
| Mrc1_3 | 0,037 | 0,006 | 0,038 | 0,033 | -0,064 | 0,052 |
| Mrc1_5 | -0,001 | 0,001 | 0,000 | 0,000 | -0,004 | 0,001 |
| Mrc1_6 | 0,004 | 0,002 | 0,004 | 0,006 | -0,001 | 0,013 |
| Mrc1_7 | -0,002 | 0,007 | 0,020 | 0,001 | -0,008 | 0,002 |
| Mrc1_8 | -0,057 | 0,007 | 0,019 | 0,039 | -0,021 | 0,005 |
| Mrc2_2 | 0,017 | 0,000 | 0,038 | 0, 018 | 0,009 | 0,011 |
| Mrc2_3 | 0,003 | 0,001 | 0,003 | 0,001 | 0,010 | 0,002 |
| Mrc2_5 | -0,002 | 0,000 | 0,002 | 0,002 | -0,005 | 0,003 |
| Mrc2_6 | 0,011 | 0,007 | 0,006 | 0,002 | -0,019 | 0,014 |
| Mrc2_7 | 0,002 | 0,002 | 0,002 | 0,001 | 0,000 | 0,001 |
| Mrc2_8 | 0,000 | 0,001 | 0,003 | 0,002 | -0,001 | 0,007 |
| Ncan | -0,090 | 0,015 | 0,279 | 0,243 | -0,589 | 0,158 |
| Olr1 | 0,083 | 0,006 | 0,379 | 0,191 | 0,116 | 0,077 |
| Pkd1 | 0,000 | 0,001 | 0,000 | 0,000 | -0,010 | 0,002 |
| Pkd1l2 | 0,001 | 0,000 | 0,001 | 0,000 | -0,002 | 0,004 |
| Pkd1l3 | 0,046 | 0,000 | -0,001 | 0,014 | 0,017 | 0,061 |
| Pla2r1_1 | 0,000 | 0,000 | 0,000 | 0,000 | -0,066 | 0,016 |
| Pla2r1_2 | 0,001 | 0,001 | 0,011 | 0,006 | -0,019 | 0,007 |
| Pla2r1_4 | 0,063 | 0,045 | 0,053 | 0,020 | 0,088 | 0,046 |
| Pla2r1_5 | 0,029 | 0,003 | 0,036 | 0,014 | 0,065 | 0,017 |
| Pla2r1_7 | 0,002 | 0,000 | 0,000 | 0,001 | 0,000 | 0,003 |
| Pla2r1_8 | -0,002 | 0,001 | 0,019 | 0,013 | -0,005 | 0,004 |
| Prg2 | 0,009 | 0,000 | 0,001 | 0,001 | -0,014 | 0,008 |
| Prg3 | -0,271 | 0,047 | -0,057 | 0,002 | -0,298 | 0,094 |
| Reg1 | 1,554 | 0,168 | 0,009 | 0,012 | -0,073 | 0,018 |
| Reg2 | 0,056 | 0,025 | 0,026 | 0,030 | -0,042 | 0,005 |
| Reg3a | 0,006 | 0,000 | 0,039 | 0,039 | -0,132 | 0,025 |
| Reg3b | 0,005 | 0,003 | 0,004 | 0,002 | -0,009 | 0,002 |
| Reg3d | 0,019 | 0,001 | 0,019 | 0,004 | 0,152 | 0,015 |
| Reg3g | -0,009 | 0,005 | 0,017 | 0,008 | -0,014 | 0,003 |
| Sele | 0,001 | 0,002 | 0,000 | 0,002 | 0,000 | 0,004 |
| Sell | -0,016 | 0,007 | -0,041 | 0,012 | -0,230 | 0,071 |
| Selp | 0,014 | 0,000 | 0,007 | 0,002 | 0,000 | 0,023 |
| Sftpa1 | 0,005 | 0,002 | 0,002 | 0,004 | -0,092 | 0,036 |
| Sftpd | 0,031 | 0,001 | 0,002 | 0,002 | 0,016 | 0,002 |
| Siglec1 | 0,000 | 0,001 | 0,000 | 0,001 | -0,002 | 0,004 |
| Siglec10 | 0,046 | 0,006 | 0,015 | 0,005 | 0,299 | 0,043 |
| Siglec12 | 0,046 | 0,019 | -0,021 | 0,009 | -0,105 | 0,010 |
| Siglec15 | 0,008 | 0,000 | 0,001 | 0,003 | 0,011 | 0,012 |
| Siglec5 | 0,041 | 0,002 | 0,019 | 0,007 | 0,146 | 0,032 |
| Siglech | 0,003 | 0,000 | 0,000 | 0,000 | -0,003 | 0,004 |
| Thbd | 0,003 | 0,000 | 0,000 | 0,000 | 0,001 | 0,002 |
| Vcan | 0,064 | 0,003 | 0,052 | 0,022 | 0,332 | 0,113 |

Based on the robust *N*-glycan dependent Spike binding, we focused our further studies on CD209c and Clec4g. We first used surface plasmon resonance (SPR) to determine the kinetic and equilibrium binding constants of these lectins to the trimeric Spike. The resulting experimental binding curves were fitted to the "bivalent analyte model" (Traxler et al. (2017) Angew Chem Int Ed Engl 56, 15755-15759) which assumes two-step binding of the lectin dimers to adjacent immobilized Spike trimer binding sites (Fig. 2E, F). From these fits, we computed the kinetic association (k_{a,1}), kinetic dissociation (k_{d,1}), and equilibrium dissociation (K_{d,1}) binding constants of single lectin bonds (Table 3). The equilibrium dissociations (K_{d,1}) values were 1.6 µM and 1.0 µM for Clec4g and CD209c, respectively.

**Table 3. Values computed for surface plasmon resonance (SPR) and atomic force microscopy (AFM). SPR. Kinetic association (k_{a,1}), kinetic dissociation (k_{d,1}), and equilibrium dissociation (K_{d,1}) constants of the first binding step fitted from the bivalent analyte model, assuming two-step binding and dissociation of the lectins to adjacent immobilized Spike trimer binding sites under spontaneous thermodynamic energy barriers; no reasonable fit was obtained with the simple 1:1 binding model. AFM. Kinetic off-rate constants (k_{off}) and lengths of dissociation paths (x_{β}) of single lectin bonds, originating from force-induced unbinding in single molecule force spectroscopy (SMFS) experiments and computed using Evans's model (Bell (1978) Science 200, 618-627; Ev-ans and Ritchie (1997) Biophys J 72, 1541-1555), assuming a sharp single dissociation energy barrier.**

| | **SPR** | | | **AFM** | |
|---|---|---|---|---|---|
| | **k_{a,1} [M⁻¹s⁻¹]** | **k_{d,1} [s⁻¹]** | **K_{d,1} [M]** | **k_{off} [s⁻¹]** | **x_{□} [nm]** |
| **Clec4g** | 6,17 x 10⁴ | 0,0997 | 1,62 x 10⁻⁶ | 0.037 ± 0.007 | 0.55 ± 0.02 |
| **CD209c** | 1,61 x 10⁴ | 0,0159 | 0,988 x 10⁻⁶ | 0.041 ± 0.025 | 0.76 ± 0.03 |
| **hCLEC4G** | 7,77 x 10⁴ | 0,0201 | 0,259 x 10⁻⁶ | 0.007 ± 0.0004 | 0.64 ± 0.55 |
| **hCD209** | 1,32 x 10⁴ | 0,0316 | 2,39 x 10⁻⁶ | 0.008 ± 0.001 | 0.73 ± 0.14 |

### Example 17: Multiple CD209c and Clec4g molecules bind simultaneously to SARS-CoV-2 Spike and form compact complexes.

To study Spike binding of these two lectins at the single-molecule level, we used atomic force microscopy (AFM) and performed single molecule force spectroscopy (SMFS) experiments. To this end, we coupled trimeric Spike to the tip of the AFM cantilever and performed single-molecule force measurements (Hinter-dorfer et al. (1996) Proc Natl Acad Sci USA 93, 3477-3481), by moving the Spike trimer-coupled tip towards the surface-bound lectins to allow for bond formations (Fig. 3A). Unbinding was accomplished by pulling on the bonds, which resulted in characteristic downward deflection signals of the cantilever, whenever a bond was ruptured (Fig. 3B). The magnitude of these vertical jumps reflects the unbinding forces, which were of typical strengths for specific molecular interactions (Rankl et al. (2008) Proc Natl Acad Sci USA 105, 17778-17783). Using this method (Rankl et al., supra; Zhu et al. (2010) Nat Nanotechnol 5, 788-791), we quantified unbinding forces (Fig. 3C) and calculated the binding probability and the number of bond ruptures between CD209c or Clec4g and trimeric Spike (Fig. 3D). Both lectins showed a very high binding probability and could establish up to 3 strong bonds with accumulating interaction force strengths reaching 150 pN in total with trimeric Spike (Fig. 3C), with the preference of single and dual bonds (Fig. 3C, 3D, fig. 7A, B, Table 3). Of note, multi-bond formation leads to stable complex formation, in which the number of formed bonds enhances the overall interaction strength and dynamic stability of the complexes. To assess dynamic interactions between single molecules of trimeric Spike and the lectins in real time we used high-speed AFM (Kodera et al. (2010) Nature 468, 72-76). Addition of Clec4g and CD209c led to a volume increase of the lectin/Spike complex in comparison to the trimeric Spike alone; based on the volumes we could calculate that on average 3.2 molecules of Clec4g and 5.2 molecules of CD209c were bound to one Spike trimer (Fig. 3E, fig. 7C-E). These data show, in real-time, at single molecule resolution, that mouse Clec4g and CD209c can directly associate with trimeric Spike.

### Example 18: The human lectins CD209 and CLEC4G are high affinity receptors for SARS-CoV-2 Spike.

Having characterized binding of murine CD209 and Clec4g to Spike, we next assessed whether their closest human homologues, namely human CD209 (hCD209), and human CD299 (hCD299) for murine CD209c, and human CLEC4G (hCLEC4G) for mouse Clec4g, can also bind to full-length trimeric Spike of SARS-CoV-2. hCD209, hCD299, and hCLEC4G indeed exhibited binding to Spike (Fig. 4A), demonstrating conserved substrate specificities. The binding of these human lectins was again independent of Spike folding and abrogated by *N*-glycan removal (Fig. 4A, B). SPR measurements of hCLEC4G and hCD209 bond formation to Spike showed equilibrium dissociation (K_{d,1}) values of 0.3 µM and 2.4 µM, respectively, in which the high affinity of hCLEC4G is mainly contributed by its rapid kinetic association rate constant (Fig. 4C, D and Table 3). In addition, hCD209 and hCLEC4G showed a high binding probability by AFM with the formation of up to 3 bonds per trimeric Spike (fig. 8A-C). When we monitored the dynamic interactions of the lectins with the Spike using high speed AFM, we observed binding of - on average - 3.5 hCLEC4G and 3.6 hCD209 molecules per trimeric Spike (Fig. 4E and fig. 8D-F). In summary, our data using ELISA, SMFS, surface plasmon resonance, and high-speed atomic force microscopy show that the human lectins CLEC4G and CD209 can bind to trimeric Spike of SARS-CoV-2, in which the overall interaction strength and dynamic stability leads to compact complex formation.

### Example 19: CLEC4G sterically interferes with Spike/ACE2 interaction.

We next 3D modelled binding of CLEC4G and CD209 to the candidate glycosylation sites present on Spike and how such attachment might relate to the binding of the trimeric Spike protein to its receptor ACE2. hCD209 is known to bind with high affinity to oligo-mannose structures (Guo et al., supra). The N234 glycosylation site is the only site within the Spike that carries exclusively oligo-mannose glycans with up to 9 mannose residues (Fig. 1C, Fig. 10). 3D modelling revealed that the oligo-mannose glycans on N234 are accessible for CD209 binding on all 3 monomers comprising the trimeric Spike (Fig. 5A and fig. 9A, B). Superimposition of ACE2 interacting with the RBD and CD209 binding to N234, showed that the CD209 binding occurs at the lateral interface of Spike, distant from the RBD (Fig. 5A). Human CLEC4G and mouse Clec4g have a high affinity for complex *N*-glycans terminating with GlcNAc. The *N*-glycan at N343, located within the RBD, is the glycosylation site most abundantly decorated with terminal GlcNAc in Spike (Fig. 1C, Fig. 10). The terminal GlcNAc glycans on position N343 are accessible for hCLEC4G binding on all 3 Spike monomers, but in contrast to CD209, hCLEC4G binding interferes with the ACE2/RBD interaction (Fig. 5A, fig. 9C, D). Similarly, modelling murine Clec4g, having the same ligands as hCLEC4G (Pipirou et al. (2011) Glycobiology 21, 806-812), binding to the N343 glycan site, also predicted interference with the ACE2/RBD interaction (fig. 9E). Thus, whereas hCD209 is not predicted to interfere with ACE2/RBD binding, murine Clec4g and human CLEC4G binding to the N343 glycan impedes Spike binding to ACE2.

### Example 20: CD209c and CLEC4G block SARS-CoV-2 infection.

To test our structural models experimentally, we assessed whether these lectins could interfere with Spike binding to the surface of Vero E6 cells, a frequently used SARS-CoV-2 infection model (Monteil et al., supra). To determine this, we set-up an AFM based method, measuring spike binding activity on Vero E6 cells. Strikingly, as predicted by the structural modelling, we found that hCLEC4G, but not hCD209, significantly interfered with the binding of trimeric Spike to the Vero E6 cell surface (Fig. 5B). Similarly, mouse Clec4g, but not murine CD209c, interfered with Spike binding to Vero E6 cells (Fig. 5C). Finally, we tested the ability of these lectins to reduce the infectivity of SARS-CoV-2. Murine Clec4g significantly reduced SARS-CoV-2 infection of Vero E6 cells (Fig. 5D). SARS-CoV-2 infections of Vero E6 were also reduced by murine CD209c (Fig. 5D), indicating that glycosylation sites outside the ACE2/RBD interface are also important for SARS-CoV-2 infectivity. Finally, hCLEC4G also significantly reduced SARS-CoV-2 infection of Vero E6 cells (Fig. 5E). These data uncover that the lectins CLEC4G and CD209c can interfere with SARS-CoV-2 infections.

**Discussion:** The examples show an unbiased screening of a comprehensive mammalian lectin library for therapeutic agent identification. As an example, use of the library, potent SARS-CoV-2 Spike binding, identifying mouse CD209c and Clec4g, as well as their human homologs hCD209 and hCLEC4G, as N-glycan dependent Spike receptors were identified. hCD209 has been identified as candidate receptor for SARS-CoV-2 by other groups, and other lectins have been also implicated in cellular interactions with Spike (Gao et al. (2020) supra; Thépaut et al. (2020) bioRxiv doi: 10.1101/2020.08.09.242917). CLEC4G has been reported to associate with SARS-CoV (Gramberg et al. (2005) Virology 340, 224-236), but has not been implicated in SARS-CoV-2 infections and not as a therapeutic target.

High-speed atomic force microscopy allowed us to directly observe Spike/lectin interactions in real time. Our high-speed AFM data showed that Spike/CLEC4G formed more rigid clusters with lower conformational flexibility as compared to the Spike/CD209 complexes in equilibrium conditions. This is in accordance with faster association rates and shorter dissociation paths of CLEC4G as compared to CD209. The experimentally observed association of 3-4 CLEC4G molecules with one molecule of the trimeric spike indicates the formation of high affinity bonds to 1-2 glycosylation sites per monomeric subunit of the trimeric Spike.

Since glycosylation is not a template driven process, but rather depends on the coordinated action of many glycosyltrans-ferases and glycosidases, each glycosylation site can - within some boundaries - carry a range of glycans. As a consequence, the 3 monomers of Spike can harbor different glycans on the same glycosylation site on different Spike proteins. We identified N343 as the one glycosylation site that is almost exclusively covered with GlcNAc terminated glycans, the ligands of CLEC4G. Given its localization in the RBD and its abundant decoration with potential CLEC4G ligands, we hypothesized that CLEC4G binding interferes with the RBD-ACE2 interaction. Indeed, we found that murine Clec4g and human CLEC4G, acting as a multi-valent effective inhibitor (K_{I} ∼ 35-70 nM), can functionally impede with Spike binding to host cell membranes, thereby providing a rationale how this lectin can affect SARS-CoV-2 infections. In support of our data, it has recently been reported that a N343 glycosylation mutant exhibits reduced infectivity using pseudo-typed viruses (Li et al. (2020) Cell 182(5), 1284-1294.e9). While CLEC4G can interfere with RBD-ACE2 binding, CD209 does apparently not associate with glycans near the RBD and hence does not block Spike binding to cells. This is in agreement to its proposed high affinity oligo-mannosidic ligands, presented at N234, which is localized at a distance to the RBD-ACE2 interface. Whether CD209 interferes with proteolytic cleavage or alpha-helical interactions of Spike can be explored. Our data identifies the key glycan ligands for CLEG4G and CD209. Binding to other glycosylation sites of lower affinities is not excluded.

Lectins play critical roles in multiple aspects of biology such as immune responses, vascular functions, or as endogenous receptors for various human pathogens. Hence, our library containing a selection of 143 lectins or more allows to comprehensively probe and map glycan structures on viruses, bacteria or fungi, as well as during development or on cancer cells, providing novel insights on the role of lectin-glycosylation interactions in infections, basic biology, and disease. For instance, CD209 is expressed by antigen presenting dendritic cells, as well as inflammatory macrophages and is known to bind to a variety of pathogens, like HIV and Ebola, but also Mycobacterium tuberculosis or Candida albicans. CLEC4G is strongly expressed in liver and lymph node sinusoidal endothelial cells and can also be found on stimulated dendritic cells and macrophages. CD299, one of the two homologues of mouse CD209c, which we also identified to possess Spike binding ability, is co-expressed with CLEC4G on liver and lymph node sinusoidal endothelial cells. Sinusoidal endothelial cells are important in the innate immune response, by acting as scavengers for pathogens as well as antigen cross-presenting cells. Thus, lectin binding to Spike allows to couple SARS-CoV-2 infections to antiviral immunity. Since viral protein glycosylation depends on the glycosylation machineries of the infected cells which assemble viral particles, slight changes in glycosylation might explain differences in anti-viral immunity and possibly severity of the disease, with critical implications for vaccine designs. Moreover, Spike-binding lectins could enhance viral entry in tissues with low ACE2 expression, thus extending the organ tropism of SARS-CoV-2.

## Claims

1. A polypeptide or nucleic acid for use in a method of treating a coronavirus infection comprising administering a polypeptide comprising a carbohydrate recognition domain of CLEC4G or a nucleic acid encoding said polypeptide to a patient suffering from an infection with a coronavirus expressing a SARS-CoV-2 Spike protein.

2. The polypeptide or nucleic acid for use according to claim 1 wherein the polypeptide comprises CLEC4G.

3. The polypeptide or nucleic acid for use according to claim 1 or 2, wherein the polypeptide comprises an immunoglobulin domain, preferably an antibody CH1, CH2 or CH3 domain.

4. The polypeptide or nucleic acid for use according to claim 3, wherein the polypeptide comprises an antibody Fc fragment.

5. The polypeptide or nucleic acid for use according to any one of claims 1 to 4, wherein the polypeptide is a dimer.

6. The polypeptide or nucleic acid for use according to any one of claims 1 to 5, wherein the CLEC4G is human CLEC4G and the patient is a human.

7. The polypeptide or nucleic acid for use according to any one of claims 1 to 6, wherein the polypeptide lacks a membrane domain and/or wherein the polypeptide comprises 50 to 250, preferably 100 to 180, amino acids of CLEC4G.

8. The polypeptide or nucleic acid for use according to any one of claims 1 to 7, wherein the polypeptide or nucleic acid is administered by inhalation, intravenous, intraarterial, intramuscular, intravascular, intraperitoneal, sub-cutaneous or oral administration.

9. The polypeptide or nucleic acid for use according to any one of claims 1 to 8, wherein the infection is an infection with SARS-CoV-2.

10. The polypeptide or nucleic acid for use according to any one of claims 1 to 8, wherein the SARS-CoV-2 Spike protein has a sequence identity of at least 85%, preferably at least 90%, more preferred at least 95%, to SEQ ID NO: 1.

11. A library of at least 10 different polypeptides, each polypeptide comprising a different carbohydrate recognition domain of a lectin and an immunoglobulin domain.

12. The library according to claim 13, wherein the lectins are selected from C-type lectins, galectins and siglecs.

13. The library according to claim 11 or 12, wherein the polypeptides lack a lectin membrane domain and/or wherein the polypeptide comprises 50 to 250, preferably 100 to 180, amino acids in length of CLEC4G.

14. A set of nucleic acids encoding the polypeptides of a library of any one of claims 13 to 15.

15. A method of identifying or screening a lectin candidate that binds to a carbohydrate-containing target of interest comprising contacting the target with the polypeptides of a library of any one of claims 11 to 13, and detecting polypeptides bound to said target.

16. The method of claim 15 comprising immobilization of the target and detecting polypeptides that are immobilized through binding the immobilized target; and/or wherein the polypeptides are labelled by binding a labelled immunoglobulin-binding moiety to the polypeptides' immunoglobulin domain.
